# EUROPEAN PATENT SPECIFICATION

(11) **EP 1 945 657 B1**
(45) Date of publication and mention of the grant of the patent: **26.10.2011**
(21) Application number: 06846186.2
(22) Date of filing: 30.10.2006
(51) Int. Cl.: C07K 14/00

(54) **Tlr3 glycosylation site muteins and methods of use**
Tlr3-Glycosylierungsstandortmuteine und Verwendungsverfahren dafür
Mutéines des sites de glycosylation de Tlr3 et leurs procédés d'utilisation

(30) Priority: 28.10.2005 US 731105 P
(43) Date of publication of application: 23.07.2008
(73) Proprietor: Janssen Biotech, Inc, Horsham, PA 19044 (US)
(72) Inventor: DUFFY, Karen, E., Trappe, PA 19426 (US); CUNNINGHAM, Mark, Kennett Square, PA 19348 (US); MBOW, M., Lamine, King of Prussia, PA 19406 (US); SARISKY, Robert, T., Landsdale, PA 19446 (US)
(74) Representative: Marshall, Cameron John
(86) International application number: PCT/US2006/060357
(87) International publication number: WO 2007/051201

(56) References cited:
- US-A- 5 243 540
- US-B1- 6 900 016
- DATABASE EMBL [Online] 24 November 2004 (2004-11-24), "Bos taurus mRNA for Toll-like-receptor 3 (tlr3 gene)" XP002522760 retrieved from EBI accession no. EMBL:AJ812026 Database accession no. AJ812026
- DE BOUTEILLER ODETTE ET AL: "Recognition of double-stranded RNA by human toll-like receptor 3 and downstream receptor signaling requires multimerization and an acidic pH." THE JOURNAL OF BIOLOGICAL CHEMISTRY 18 NOV 2005, vol. 280, no. 46, 6 September 2005 (2005-09-06), pages 38133-38145, XP002522758 ISSN: 0021-9258
- BELL JESSICA K ET AL: "The molecular structure of the Toll-like receptor 3 ligand-binding domain." PROCEEDINGS OF THE NATIONAL ACADEMY OF SCIENCES OF THE UNITED STATES OF AMERICA 2 AUG 2005, vol. 102, no. 31, 2 August 2005 (2005-08-02), pages 10976-10980, XP002522759 ISSN: 0027-8424
- SANGHAVI SONALI K ET AL: "Increased expression of TLR3 in lymph nodes during simian immunodeficiency virus infection: implications for inflammation and immunodeficiency.", JOURNAL OF IMMUNOLOGY (BALTIMORE, MD. : 1950) 15 OCT 2005 LNKD- PUBMED:16210637, vol. 175, no. 8, 15 October 2005 (2005-10-15), pages 5314-5323, ISSN: 0022-1767

### SEQUENCE LISTING

<110> CENTOCOR, INC.
<120> TLR3 GLYCOSYLATION SITE MUTEINS AND METHODS OF USE
<130> CEN5120PCT
<140> TO BE ASSIGNED
   <141> 2006-10-30
<140> 60/731,105
   <141> 2005-10-28
<160> 20
<170> FastSEQ for Windows Version 4.0
<210> 1
   <211> 2710
   <212> DNA
   <213> Homo sapiens
<400> 1
   atgagacaga ctttgccttg tatctacttt tgggggggcc ttttgccctt tgggatgctg 60
   tgtgcatcct ccaccaccaa gtgcactgtt agccatgaag ttgctgactg cagccacctg 120
   aagttgactc aggtacccga tgatctaccc acaaacataa cagtgttgaa ccttacccat 180
   aatcaactca gaagattacc agccgccaac ttcacaaggt atagccagct aactagcttg 240
   gatgtaggat ttaacaccat ctcaaaactg gagccagaat tgtgccagaa acttcccatg 300
   ttaaaagttt tgaacctcca gcacaatgag ctatctcaac tttctgataa aacctttgcc 360
   ttctgcacga atttgactga actccatctc atgtccaact caatccagaa aattaaaaat 420
   aatccctttg tcaagcagaa gaatttaatc acattagatc tgtctcataa tggcttgtca 480
   tctacaaaat taggaactca ggttcagctg gaaaatctcc aagagcttct attatcaaac 540
   aataaaattc aagcgctaaa aagtgaagaa ctggatatct ttgccaattc atctttaaaa 600
   aaattagagt tgtcatcgaa tcaaattaaa gagttttctc cagggtgttt tcacgcaatt 660
   ggaagattat ttggcctctt tctgaacaat gtccagctgg gtcccagcct tacagagaag 720
   ctatgtttgg aattagcaaa cacaagcatt cggaatctgt ctctgagtaa cagccagctg 780
   tccaccacca gcaatacaac tttcttggga ctaaagtgga caaatctcac tatgctcgat 840
   ctttcctaca acaacttaaa tgtggttggt aacgattcct ttgcttggct tccacaacta 900
   gaatatttct tcctagagta taataatata cagcatttgt tttctcactc tttgcacggg 960
   cttttcaatg tgaggtacct gaatttgaaa cggtctttta ctaaacaaag tatttccctt 1020
   gcctcactcc ccaagattga tgatttttct tttcagtggc taaaatgttt ggagcacctt 1080
   aacatggaag ataatgatat tccaggcata aaaagcaata tgttcacagg attgataaac 1140
   ctgaaatact taagtctatc caactccttt acaagtttgc gaactttgac aaatgaaaca 1200
   tttgtatcac ttgctcattc tcccttacac atactcaacc taaccaagaa taaaatctca 1260
   aaaatagaga gtgatgcttt ctcttggttg ggccacctag aagtacttga cctgggcctt 1320
   aatgaaattg ggcaagaact cacaggccag gaatggagag gtctagaaaa tattttcgaa 1380
   atctatcttt cctacaacaa gtacctgcag ctgactagga actcctttgc cttggtccca 1440
   agccttcaac gactgatgct ccgaagggtg gcccttaaaa atgtggatag ctctccttca 1500
   ccattccagc ctcttcgtaa cttgaccatt ctggatctaa gcaacaacaa catagccaac 1560
   ataaatgatg acatgttgga gggtcttgag aaactagaaa ttctcgattt gcagcataac 1620
   aacttagcac ggctctggaa acacgcaaac cctggtggtc ccatttattt cctaaagggt 1680
   ctgtctcacc tccacatcct taacttggag tccaacggct ttgacgagat cccagttgag 1740
   gtcttcaagg atttatttga actaaagatc atcgatttag gattgaataa tttaaacaca 1800
   cttccagcat ctgtctttaa taatcaggtg tctctaaagt cattgaacct tcagaagaat 1860
   ctcataacat ccgttgagaa gaaggttttc gggccagctt tcaggaacct gactgagtta 1920
   gatatgcgct ttaatccctt tgattgcacg tgtgaaagta ttgcctggtt tgttaattgg 1980
   attaacgaga cccataccaa catccctgag ctgtcaagcc actacctttg caacactcca 2040
   cctcactatc atgggttccc agtgagactt tttgatacat catcttgcaa agacagtgcc 2100
   ccctttgaac tctttttcat gatcaatacc agtatcctgt tgatttttat ctttattgta 2160
   cttctcatcc actttgaggg ctggaggata tctttttatt ggaatgtttc agtacatcga 2220
   gttcttggtt tcaaagaaat agacagacag acagaacagt ttgaatatgc agcatatata 2280
   attcatgcct ataaagataa ggattgggtc tgggaacatt tctcttcaat ggaaaaggaa 2340
   gaccaatctc tcaaattttg tctggaagaa agggactttg aggcgggtgt ttttgaacta 2400
   gaagcaattg ttaacagcat caaaagaagc agaaaaatta tttttgttat aacacaccat 2460
   ctattaaaag acccattatg caaaagattc aaggtacatc atgcagttca acaagctatt 2520
   gaacaaaatc tggattccat tatattggtt ttccttgagg agattccaga ttataaactg 2580
   aaccatgcac tctgtttgcg aagaggaatg tttaaatctc actgcatctt gaactggcca 2640
   gttcagaaag aacggatagg tgcctttcgt cataaattgc aagtagcact tggatccaaa 2700
   aactctgtac                                                        2710
<210> 2
   <211> 904
   <212> PRT
   <213> Homo sapiens
<400> 2
   Met Arg Gln Thr Leu Pro Cys Ile Tyr Phe Trp Gly Gly Leu Leu Pro
    1               5                  10                  15
   Phe Gly Met Leu Cys Ala Ser Ser Thr Thr Lys Cys Thr Val Ser His
               20                  25                  30
   Glu Val Ala Asp Cys Ser His Leu Lys Leu Thr Gln Val Pro Asp Asp
           35                  40                  45
   Leu Pro Thr Asn Ile Thr Val Leu Asn Leu Thr His Asn Gln Leu Arg
       50                  55                  60
   Arg Leu Pro Ala Ala Asn Phe Thr Arg Tyr Ser Gln Leu Thr Ser Leu
   65                  70                  75                  80
   Asp Val Gly Phe Asn Thr Ile Ser Lys Leu Glu Pro Glu Leu Cys Gln
                   85                  90                  95
   Lys Leu Pro Met Leu Lys Val Leu Asn Leu Gln His Asn Glu Leu Ser
               100                 105                 110
   Gln Leu Ser Asp Lys Thr Phe Ala Phe Cys Thr Asn Leu Thr Glu Leu
           115                 120                 125
   His Leu Met Ser Asn Ser Ile Gln Lys Ile Lys Asn Asn Pro Phe Val
       130                 135                 140
   Lys Gln Lys Asn Leu Ile Thr Leu Asp Leu Ser His Asn Gly Leu Ser
   145                 150                 155                 160
   Ser Thr Lys Leu Gly Thr Gln Val Gln Leu Glu Asn Leu Gln Glu Leu
                   165                 170                 175
   Leu Leu Ser Asn Asn Lys Ile Gln Ala Leu Lys Ser Glu Glu Leu Asp
               180                 185                 190
   Ile Phe Ala Asn Ser Ser Leu Lys Lys Leu Glu Leu Ser Ser Asn Gln
           195                 200                 205
   Ile Lys Glu Phe Ser Pro Gly Cys Phe His Ala Ile Gly Arg Leu Phe
       210                 215                 220
   Gly Leu Phe Leu Asn Asn Val Gln Leu Gly Pro Ser Leu Thr Glu Lys
   225                 230                 235                 240
   Leu Cys Leu Glu Leu Ala Asn Thr Ser Ile Arg Asn Leu Ser Leu Ser
                   245                 250                 255
   Asn Ser Gln Leu Ser Thr Thr Ser Asn Thr Thr Phe Leu Gly Leu Lys
               260                 265                 270
   Trp Thr Asn Leu Thr Met Leu Asp Leu Ser Tyr Asn Asn Leu Asn Val
           275                 280                 285
   Val Gly Asn Asp Ser Phe Ala Trp Leu Pro Gln Leu Glu Tyr Phe Phe
       290                 295                 300
   Leu Glu Tyr Asn Asn Ile Gln His Leu Phe Ser His Ser Leu His Gly
   305                 310                 315                 320
   Leu Phe Asn Val Arg Tyr Leu Asn Leu Lys Arg Ser Phe Thr Lys Gln
                   325                 330                 335
   Ser Ile Ser Leu Ala Ser Leu Pro Lys Ile Asp Asp Phe Ser Phe Gln
               340                 345                 350
   Trp Leu Lys Cys Leu Glu His Leu Asn Met Glu Asp Asn Asp Ile Pro
           355                 360                 365
   Gly Ile Lys Ser Asn Met Phe Thr Gly Leu Ile Asn Leu Lys Tyr Leu
       370                 375                 380
   Ser Leu Ser Asn Ser Phe Thr Ser Leu Arg Thr Leu Thr Asn Glu Thr
   385                 390                 395                 400
   Phe Val Ser Leu Ala His Ser Pro Leu His Ile Leu Asn Leu Thr Lys
                   405                 410                 415
   Asn Lys Ile Ser Lys Ile Glu Ser Asp Ala Phe Ser Trp Leu Gly His
               420                 425                 430
   Leu Glu Val Leu Asp Leu Gly Leu Asn Glu Ile Gly Gln Glu Leu Thr
           435                 440                 445
   Gly Gln Glu Trp Arg Gly Leu Glu Asn Ile Phe Glu Ile Tyr Leu Ser
       450                 455                 460
   Tyr Asn Lys Tyr Leu Gln Leu Thr Arg Asn Ser Phe Ala Leu Val Pro
   465                 470                 475                 480
   Ser Leu Gln Arg Leu Met Leu Arg Arg Val Ala Leu Lys Asn Val Asp
                   485                 490                 495
   Ser Ser Pro Ser Pro Phe Gln Pro Leu Arg Asn Leu Thr Ile Leu Asp
               500                 505                 510
   Leu Ser Asn Asn Asn Ile Ala Asn Ile Asn Asp Asp Met Leu Glu Gly
           515                 520                 525
   Leu Glu Lys Leu Glu Ile Leu Asp Leu Gln His Asn Asn Leu Ala Arg
       530                 535                 540
   Leu Trp Lys His Ala Asn Pro Gly Gly Pro Ile Tyr Phe Leu Lys Gly
   545                 550                 555                 560
   Leu Ser His Leu His Ile Leu Asn Leu Glu Ser Asn Gly Phe Asp Glu
                   565                 570                 575
   Ile Pro Val Glu Val Phe Lys Asp Leu Phe Glu Leu Lys Ile Ile Asp
               580                 585                 590
   Leu Gly Leu Asn Asn Leu Asn Thr Leu Pro Ala Ser Val Phe Asn Asn
           595                 600                 605
   Gln Val Ser Leu Lys Ser Leu Asn Leu Gln Lys Asn Leu Ile Thr Ser
       610                 615                 620
   Val Glu Lys Lys Val Phe Gly Pro Ala Phe Arg Asn Leu Thr Glu Leu
   625                 630                 635                 640
   Asp Met Arg Phe Asn Pro Phe Asp Cys Thr Cys Glu Ser Ile Ala Trp
                   645                 650                 655
   Phe Val Asn Trp Ile Asn Glu Thr His Thr Asn Ile Pro Glu Leu Ser
               660                 665                 670
   Ser His Tyr Leu Cys Asn Thr Pro Pro His Tyr His Gly Phe Pro Val
           675                 680                 685
   Arg Leu Phe Asp Thr Ser Ser Cys Lys Asp Ser Ala Pro Phe Glu Leu
       690                 695                 700
   Phe Phe Met Ile Asn Thr Ser Ile Leu Leu Ile Phe Ile Phe Ile Val
   705                 710                 715                 720
   Leu Leu Ile His Phe Glu Gly Trp Arg Ile Ser Phe Tyr Trp Asn Val
                   725                 730                 735
   Ser Val His Arg Val Leu Gly Phe Lys Glu Ile Asp Arg Gln Thr Glu
               740                 745                 750
   Gln Phe Glu Tyr Ala Ala Tyr Ile Ile His Ala Tyr Lys Asp Lys Asp
           755                 760                 765
   Trp Val Trp Glu His Phe Ser Ser Met Glu Lys Glu Asp Gln Ser Leu
       770                 775                 780
   Lys Phe Cys Leu Glu Glu Arg Asp Phe Glu Ala Gly Val Phe Glu Leu
   785                 790                 795                 800
   Glu Ala Ile Val Asn Ser Ile Lys Arg Ser Arg Lys Ile Ile Phe Val
                   805                 810                 815
   Ile Thr His His Leu Leu Lys Asp Pro Leu Cys Lys Arg Phe Lys Val
               820                 825                 830
   His His Ala Val Gln Gln Ala Ile Glu Gln Asn Leu Asp Ser Ile Ile
           835                 840                 845
   Leu Val Phe Leu Glu Glu Ile Pro Asp Tyr Lys Leu Asn His Ala Leu
       850                 855                 860
   Cys Leu Arg Arg Gly Met Phe Lys Ser His Cys Ile Leu Asn Trp Pro
   865                 870                 875                 880
   Val Gln Lys Glu Arg Ile Gly Ala Phe Arg His Lys Leu Gln Val Ala
                   885                 890                 895
   Leu Gly Ser Lys Asn Ser Val His
               900
<210> 3
   <211> 2109
   <212> DNA
   <213> Homo sapiens
<400> 3
   atgagacaga ctttgccttg tatctacttt tgggggggcc ttttgccctt tgggatgctg 60
   tgtgcatcct ccaccaccaa gtgcactgtt agccatgaag ttgctgactg cagccacctg 120
   aagttgactc aggtacccga tgatctaccc acaaacataa cagtgttgaa ccttacccat 180
   aatcaactca gaagattacc agccgccaac ttcacaaggt atagccagct aactagcttg 240
   gatgtaggat ttaacaccat ctcaaaactg gagccagaat tgtgccagaa acttcccatg 300
   ttaaaagttt tgaacctcca gcacaatgag ctatctcaac tttctgataa aacctttgcc 360
   ttctgcacga atttgactga actccatctc atgtccaact caatccagaa aattaaaaat 420
   aatccctttg tcaagcagaa gaatttaatc acattagatc tgtctcataa tggcttgtca 480
   tctacaaaat taggaactca ggttcagctg gaaaatctcc aagagcttct attatcaaac 540
   aataaaattc aagcgctaaa aagtgaagaa ctggatatct ttgccaattc atctttaaaa 600
   aaattagagt tgtcatcgaa tcaaattaaa gagttttctc cagggtgttt tcacgcaatt 660
   ggaagattat ttggcctctt tctgaacaat gtccagctgg gtcccagcct tacagagaag 720
   ctatgtttgg aattagcaaa cacaagcatt cggaatctgt ctctgagtaa cagccagctg 780
   tccaccacca gcaatacaac tttcttggga ctaaagtgga caaatctcac tatgctcgat 840
   ctttcctaca acaacttaaa tgtggttggt aacgattcct ttgcttggct tccacaacta 900
   gaatatttct tcctagagta taataatata cagcatttgt tttctcactc tttgcacggg 960
   cttttcaatg tgaggtacct gaatttgaaa cggtctttta ctaaacaaag tatttccctt 1020
   gcctcactcc ccaagattga tgatttttct tttcagtggc taaaatgttt ggagcacctt 1080
   aacatggaag ataatgatat tccaggcata aaaagcaata tgttcacagg attgataaac 1140
   ctgaaatact taagtctatc caactccttt acaagtttgc gaactttgac aaatgaaaca 1200
   tttgtatcac ttgctcattc tcccttacac atactcaacc taaccaagaa taaaatctca 1260
   aaaatagaga gtgatgcttt ctcttggttg ggccacctag aagtacttga cctgggcctt 1320
   aatgaaattg ggcaagaact cacaggccag gaatggagag gtctagaaaa tattttcgaa 1380
   atctatcttt cctacaacaa gtacctgcag ctgactagga actcctttgc cttggtccca 1440
   agccttcaac gactgatgct ccgaagggtg gcccttaaaa atgtggatag ctctccttca 1500
   ccattccagc ctcttcgtaa cttgaccatt ctggatctaa gcaacaacaa catagccaac 1560
   ataaatgatg acatgttgga gggtcttgag aaactagaaa ttctcgattt gcagcataac 1620
   aacttagcac ggctctggaa acacgcaaac cctggtggtc ccatttattt cctaaagggt 1680
   ctgtctcacc tccacatcct taacttggag tccaacggct ttgacgagat cccagttgag 1740
   gtcttcaagg atttatttga actaaagatc atcgatttag gattgaataa tttaaacaca 1800
   cttccagcat ctgtctttaa taatcaggtg tctctaaagt cattgaacct tcagaagaat 1860
   ctcataacat ccgttgagaa gaaggttttc gggccagctt tcaggaacct gactgagtta 1920
   gatatgcgct ttaatccctt tgattgcacg tgtgaaagta ttgcctggtt tgttaattgg 1980
   attaacgaga cccataccaa catccctgag ctgtcaagcc actacctttg caacactcca 2040
   cctcactatc atgggttccc agtgagactt tttgatacat catcttgcaa agacagtgcc 2100
   ccctttgaa                                                         2109
<210> 4
   <211> 703
   <212> PRT
   <213> Homo sapiens
<400> 4
   Met Arg Gln Thr Leu Pro Cys Ile Tyr Phe Trp Gly Gly Leu Leu Pro
    1               5                  10                  15
   Phe Gly Met Leu Cys Ala Ser Ser Thr Thr Lys Cys Thr Val Ser His
               20                  25                  30
   Glu Val Ala Asp Cys Ser His Leu Lys Leu Thr Gln Val Pro Asp Asp
           35                  40                  45
   Leu Pro Thr Asn Ile Thr Val Leu Asn Leu Thr His Asn Gln Leu Arg
       50                  55                  60
   Arg Leu Pro Ala Ala Asn Phe Thr Arg Tyr Ser Gln Leu Thr Ser Leu
   65                  70                  75                  80
   Asp Val Gly Phe Asn Thr Ile Ser Lys Leu Glu Pro Glu Leu Cys Gln
                   85                  90                  95
   Lys Leu Pro Met Leu Lys Val Leu Asn Leu Gln His Asn Glu Leu Ser
               100                 105                 110
   Gln Leu Ser Asp Lys Thr Phe Ala Phe Cys Thr Asn Leu Thr Glu Leu
           115                 120                 125
   His Leu Met Ser Asn Ser Ile Gln Lys Ile Lys Asn Asn Pro Phe Val
       130                 135                 140
   Lys Gln Lys Asn Leu Ile Thr Leu Asp Leu Ser His Asn Gly Leu Ser
   145                 150                 155                 160
   Ser Thr Lys Leu Gly Thr Gln Val Gln Leu Glu Asn Leu Gln Glu Leu
                   165                 170                 175
   Leu Leu Ser Asn Asn Lys Ile Gln Ala Leu Lys Ser Glu Glu Leu Asp
               180                 185                 190
   Ile Phe Ala Asn Ser Ser Leu Lys Lys Leu Glu Leu Ser Ser Asn Gln
           195                 200                 205
   Ile Lys Glu Phe Ser Pro Gly Cys Phe His Ala Ile Gly Arg Leu Phe
       210                 215                 220
   Gly Leu Phe Leu Asn Asn Val Gln Leu Gly Pro Ser Leu Thr Glu Lys
   225                 230                 235                 240
   Leu Cys Leu Glu Leu Ala Asn Thr Ser Ile Arg Asn Leu Ser Leu Ser
                   245                 250                 255
   Asn Ser Gln Leu Ser Thr Thr Ser Asn Thr Thr Phe Leu Gly Leu Lys
               260                 265                 270
   Trp Thr Asn Leu Thr Met Leu Asp Leu Ser Tyr Asn Asn Leu Asn Val
           275                 280                 285
   Val Gly Asn Asp Ser Phe Ala Trp Leu Pro Gln Leu Glu Tyr Phe Phe
       290                 295                 300
   Leu Glu Tyr Asn Asn Ile Gln His Leu Phe Ser His Ser Leu His Gly
   305                 310                 315                 320
   Leu Phe Asn Val Arg Tyr Leu Asn Leu Lys Arg Ser Phe Thr Lys Gln
                   325                 330                 335
   Ser Ile Ser Leu Ala Ser Leu Pro Lys Ile Asp Asp Phe Ser Phe Gln
               340                 345                 350
   Trp Leu Lys Cys Leu Glu His Leu Asn Met Glu Asp Asn Asp Ile Pro
           355                 360                 365
   Gly Ile Lys Ser Asn Met Phe Thr Gly Leu Ile Asn Leu Lys Tyr Leu
       370                 375                 380
   Ser Leu Ser Asn Ser Phe Thr Ser Leu Arg Thr Leu Thr Asn Glu Thr
   385                 390                 395                 400
   Phe Val Ser Leu Ala His Ser Pro Leu His Ile Leu Asn Leu Thr Lys
                   405                 410                 415
   Asn Lys Ile Ser Lys Ile Glu Ser Asp Ala Phe Ser Trp Leu Gly His
               420                 425                 430
   Leu Glu Val Leu Asp Leu Gly Leu Asn Glu Ile Gly Gln Glu Leu Thr
           435                 440                 445
   Gly Gln Glu Trp Arg Gly Leu Glu Asn Ile Phe Glu Ile Tyr Leu Ser
       450                 455                 460
   Tyr Asn Lys Tyr Leu Gln Leu Thr Arg Asn Ser Phe Ala Leu Val Pro
   465                 470                 475                 480
   Ser Leu Gln Arg Leu Met Leu Arg Arg Val Ala Leu Lys Asn Val Asp
                   485                 490                 495
   Ser Ser Pro Ser Pro Phe Gln Pro Leu Arg Asn Leu Thr Ile Leu Asp
               500                 505                 510
   Leu Ser Asn Asn Asn Ile Ala Asn Ile Asn Asp Asp Met Leu Glu Gly
           515                 520                 525
   Leu Glu Lys Leu Glu Ile Leu Asp Leu Gln His Asn Asn Leu Ala Arg
       530                 535                 540
   Leu Trp Lys His Ala Asn Pro Gly Gly Pro Ile Tyr Phe Leu Lys Gly
   545                 550                 555                 560
   Leu Ser His Leu His Ile Leu Asn Leu Glu Ser Asn Gly Phe Asp Glu
                   565                 570                 575
   Ile Pro Val Glu Val Phe Lys Asp Leu Phe Glu Leu Lys Ile Ile Asp
               580                 585                 590
   Leu Gly Leu Asn Asn Leu Asn Thr Leu Pro Ala Ser Val Phe Asn Asn
           595                 600                 605
   Gln Val Ser Leu Lys Ser Leu Asn Leu Gln Lys Asn Leu Ile Thr Ser
       610                 615                 620
   Val Glu Lys Lys Val Phe Gly Pro Ala Phe Arg Asn Leu Thr Glu Leu
   625                 630                 635                 640
   Asp Met Arg Phe Asn Pro Phe Asp Cys Thr Cys Glu Ser Ile Ala Trp
                   645                 650                 655
   Phe Val Asn Trp Ile Asn Glu Thr His Thr Asn Ile Pro Glu Leu Ser
               660                 665                 670
   Ser His Tyr Leu Cys Asn Thr Pro Pro His Tyr His Gly Phe Pro Val
           675                 680                 685
   Arg Leu Phe Asp Thr Ser Ser Cys Lys Asp Ser Ala Pro Phe Glu
       690                 695                 700
<210> 5
   <211> 2031
   <212> DNA
   <213> Homo sapiens
<400> 5
   aagtgcactg ttagccatga agttgctgac tgcagccacc tgaagttgac tcaggtaccc 60
   gatgatctac ccacaaacat aacagtgttg aaccttaccc ataatcaact cagaagatta 120
   ccagccgcca acttcacaag gtatagccag ctaactagct tggatgtagg atttaacacc 180
   atctcaaaac tggagccaga attgtgccag aaacttccca tgttaaaagt tttgaacctc 240
   cagcacaatg agctatctca actttctgat aaaacctttg ccttctgcac gaatttgact 300
   gaactccatc tcatgtccaa ctcaatccag aaaattaaaa ataatccctt tgtcaagcag 360
   aagaatttaa tcacattaga tctgtctcat aatggcttgt catctacaaa attaggaact 420
   caggttcagc tggaaaatct ccaagagctt ctattatcaa acaataaaat tcaagcgcta 480
   aaaagtgaag aactggatat ctttgccaat tcatctttaa aaaaattaga gttgtcatcg 540
   aatcaaatta aagagttttc tccagggtgt tttcacgcaa ttggaagatt atttggcctc 600
   tttctgaaca atgtccagct gggtcccagc cttacagaga agctatgttt ggaattagca 660
   aacacaagca ttcggaatct gtctctgagt aacagccagc tgtccaccac cagcaataca 720
   actttcttgg gactaaagtg gacaaatctc actatgctcg atctttccta caacaactta 780
   aatgtggttg gtaacgattc ctttgcttgg cttccacaac tagaatattt cttcctagag 840
   tataataata tacagcattt gttttctcac tctttgcacg ggcttttcaa tgtgaggtac 900
   ctgaatttga aacggtcttt tactaaacaa agtatttccc ttgcctcact ccccaagatt 960
   gatgattttt cttttcagtg gctaaaatgt ttggagcacc ttaacatgga agataatgat 1020
   attccaggca taaaaagcaa tatgttcaca ggattgataa acctgaaata cttaagtcta 1080
   tccaactcct ttacaagttt gcgaactttg acaaatgaaa catttgtatc acttgctcat 1140
   tctcccttac acatactcaa cctaaccaag aataaaatct caaaaataga gagtgatgct 1200
   ttctcttggt tgggccacct agaagtactt gacctgggcc ttaatgaaat tgggcaagaa 1260
   ctcacaggcc aggaatggag aggtctagaa aatatttcgg aaatctatct ttcctacaac 1320
   aagtacctgc agctgactag gaactccttt gccttggtcc caagccttca acgactgatg 1380
   ctccgaaggg tggcccttaa aaatgtggat agctctcctt caccattcca gcctcttcgt 1440
   aacttgacca ttctggatct aagcaacaac aacatagcca acataaatga tgacatgttg 1500
   gagggtcttg agaaactaga aattctcgat ttgcagcata acaacttagc acggctctgg 1560
   aaacacgcaa accctggtgg tcccatttat ttcctaaagg gtctgtctca cctccacatc 1620
   cttaacttgg agtccaacgg ctttgacgag atcccagttg aggtcttcaa ggatttattt 1680
   gaactaaaga tcatcgattt aggattgaat aatttaaaca cacttccagc atctgtcttt 1740
   aataatcagg tgtctctaaa gtcattgaac cttcagaaga atctcataac atccgttgag 1800
   aagaaggttt tcgggccagc tttcaggaac ctgactgagt tagatatgcg ctttaatccc 1860
   tttgattgca cgtgtgaaag tattgcctgg tttgttaatt ggattaacga gacccatacc 1920
   aacatccctg agctgtcaag ccactacctt tgcaacactc cacctcacta tcatgggttc 1980
   ccagtgagac tttttgatac atcatcttgc aaagacagtg ccccctttga a          2031
<210> 6
   <211> 677
   <212> PRT
   <213> Homo sapiens
<400> 6
   Lys Cys Thr Val Ser His Glu Val Ala Asp Cys Ser His Leu Lys Leu
   1               5                   10                  15
   Thr Gln Val Pro Asp Asp Leu Pro Thr Asn Ile Thr Val Leu Asn Leu
               20                  25                  30
   Thr His Asn Gln Leu Arg Arg Leu Pro Ala Ala Asn Phe Thr Arg Tyr
           35                  40                  45
   Ser Gln Leu Thr Ser Leu Asp Val Gly Phe Asn Thr Ile Ser Lys Leu
       50                  55                  60
   Glu Pro Glu Leu Cys Gln Lys Leu Pro Met Leu Lys Val Leu Asn Leu
   65                  70                  75                  80
   Gln His Asn Glu Leu Ser Gln Leu Ser Asp Lys Thr Phe Ala Phe Cys
                   85                  90                  95
   Thr Asn Leu Thr Glu Leu His Leu Met Ser Asn Ser Ile Gln Lys Ile
               100                 105                 110
   Lys Asn Asn Pro Phe Val Lys Gln Lys Asn Leu Ile Thr Leu Asp Leu
           115                 120                 125
   Ser His Asn Gly Leu Ser Ser Thr Lys Leu Gly Thr Gln Val Gln Leu
       130                 135                 140
   Glu Asn Leu Gln Glu Leu Leu Leu Ser Asn Asn Lys Ile Gln Ala Leu
   145                 150                 155                 160
   Lys Ser Glu Glu Leu Asp Ile Phe Ala Asn Ser Ser Leu Lys Lys Leu
                   165                 170                 175
   Glu Leu Ser Ser Asn Gln Ile Lys Glu Phe Ser Pro Gly Cys Phe His
               180                 185                 190
   Ala Ile Gly Arg Leu Phe Gly Leu Phe Leu Asn Asn Val Gln Leu Gly
           195                 200                 205
   Pro Ser Leu Thr Glu Lys Leu Cys Leu Glu Leu Ala Asn Thr Ser Ile
       210                 215                 220
   Arg Asn Leu Ser Leu Ser Asn Ser Gln Leu Ser Thr Thr Ser Asn Thr
   225                 230                 235                 240
   Thr Phe Leu Gly Leu Lys Trp Thr Asn Leu Thr Met Leu Asp Leu Ser
                   245                 250                 255
   Tyr Asn Asn Leu Asn Val Val Gly Asn Asp Ser Phe Ala Trp Leu Pro
               260                 265                 270
   Gln Leu Glu Tyr Phe Phe Leu Glu Tyr Asn Asn Ile Gln His Leu Phe
           275                 280                 285
   Ser His Ser Leu His Gly Leu Phe Asn Val Arg Tyr Leu Asn Leu Lys
       290                 295                 300
   Arg Ser Phe Thr Lys Gln Ser Ile Ser Leu Ala Ser Leu Pro Lys Ile
   305                 310                 315                 320
   Asp Asp Phe Ser Phe Gln Trp Leu Lys Cys Leu Glu His Leu Asn Met
                   325                 330                 335
   Glu Asp Asn Asp Ile Pro Gly Ile Lys Ser Asn Met Phe Thr Gly Leu
               340                 345                 350
   Ile Asn Leu Lys Tyr Leu Ser Leu Ser Asn Ser Phe Thr Ser Leu Arg
           355                 360                 365
   Thr Leu Thr Asn Glu Thr Phe Val Ser Leu Ala His Ser Pro Leu His
       370                 375                 380
   Ile Leu Asn Leu Thr Lys Asn Lys Ile Ser Lys Ile Glu Ser Asp Ala
   385                 390                 395                 400
   Phe Ser Trp Leu Gly His Leu Glu Val Leu Asp Leu Gly Leu Asn Glu
                   405                 410                 415
   Ile Gly Gln Glu Leu Thr Gly Gln Glu Trp Arg Gly Leu Glu Asn Ile
               420                 425                 430
   Phe Glu Ile Tyr Leu Ser Tyr Asn Lys Tyr Leu Gln Leu Thr Arg Asn
           435                 440                 445
   Ser Phe Ala Leu Val Pro Ser Leu Gln Arg Leu Met Leu Arg Arg Val
       450                 455                 460
   Ala Leu Lys Asn Val Asp Ser Ser Pro Ser Pro Phe Gln Pro Leu Arg
   465                 470                 475                 480
   Asn Leu Thr Ile Leu Asp Leu Ser Asn Asn Asn Ile Ala Asn Ile Asn
                   485                 490                 495
   Asp Asp Met Leu Glu Gly Leu Glu Lys Leu Glu Ile Leu Asp Leu Gln
               500                 505                 510
   His Asn Asn Leu Ala Arg Leu Trp Lys His Ala Asn Pro Gly Gly Pro
           515                 520                 525
   Ile Tyr Phe Leu Lys Gly Leu Ser His Leu His Ile Leu Asn Leu Glu
       530                 535                 540
   Ser Asn Gly Phe Asp Glu Ile Pro Val Glu Val Phe Lys Asp Leu Phe
   545                 550                 555                 560
   Glu Leu Lys Ile Ile Asp Leu Gly Leu Asn Asn Leu Asn Thr Leu Pro
                   565                 570                 575
   Ala Ser Val Phe Asn Asn Gln Val Ser Leu Lys Ser Leu Asn Leu Gln
               580                 585                 590
   Lys Asn Leu Ile Thr Ser Val Glu Lys Lys Val Phe Gly Pro Ala Phe
           595                 600                 605
   Arg Asn Leu Thr Glu Leu Asp Met Arg Phe Asn Pro Phe Asp Cys Thr
       610                 615                 620
   Cys Glu Ser Ile Ala Trp Phe Val Asn Trp Ile Asn Glu Thr His Thr
   625                 630                 635                 640
   Asn Ile Pro Glu Leu Ser Ser His Tyr Leu Cys Asn Thr Pro Pro His
                   645                 650                 655
   Tyr His Gly Phe Pro Val Arg Leu Phe Asp Thr Ser Ser Cys Lys Asp
               660                 665                 670
   Ser Ala Pro Phe Glu
           675
<210> 7
   <211> 2031
   <212> DNA
   <213> Artificial Sequence
<220>
   <223> Artificial cDNA sequence derived from the Homo sapiens hTLR3 ECD which lacks a signal peptide and contains an alanine (A) substitution mutation at N221.
<400> 7
   aaatgcaccg tgagccatga agtggcggat tgcagccatc tgaaactgac ccaggtgccg 60
   gatgatctgc cgaccaacat taccgtgctg aacctgaccc ataaccagct gcgccgcctg 120
   ccggcggcga actttacccg ctatagccag ctgaccagcc tggatgtggg ctttaacacc 180
   attagcaaac tggaaccgga actgtgccag aaactgccga tgctgaaagt gctgaacctg 240
   cagcataacg aactgagcca gctgagcgat aaaacctttg cgttttgcac caacctgacc 300
   gaactgcatc tgatgagcaa cagcattcag aaaattaaaa acaacccgtt tgtgaaacag 360
   aaaaacctga ttaccctgga tctgagccat aacggcctga gcagcaccaa actgggcacc 420
   caggtgcagc tggaaaacct gcaggaactg ctgctgagca acaacaaaat tcaggcgctg 480
   aaaagcgaag aactggatat ttttgcgaac agcagcctga aaaaactgga actgagcagc 540
   aaccagatta aagaatttag cccgggctgc tttcatgcga ttggccgcct gtttggcctg 600
   tttctgaaca acgtgcagct gggcccgagc ctgaccgaaa aactgtgcct ggaactggcg 660
   gcgaccagca ttcgcaacct gagcctgagc aacagccagc tgagcaccac cagcaacacc 720
   acctttctgg gcctgaaatg gaccaacctg accatgctgg atctgagcta taacaacctg 780
   aacgtggtgg gcaacgatag ctttgcgtgg ctgccgcagc tggaatattt ttttctggaa 840
   tataacaaca ttcagcatct gtttagccat agcctgcatg gcctgtttaa cgtgcgctat 900
   ctgaacctga aacgcagctt taccaaacag agcattagcc tggcgagcct gccgaaaatt 960
   gatgatttta gctttcagtg gctgaaatgc ctggaacatc tgaacatgga agataacgat 1020
   attccgggca ttaaaagcaa catgtttacc ggcctgatta acctgaaata tctgagcctg 1080
   agcaacagct ttaccagcct gcgcaccctg accaacgaaa cctttgtgag cctggcgcat 1140
   agcccgctgc atattctgaa cctgaccaaa aacaaaatta gcaaaattga aagcgatgcg 1200
   tttagctggc tgggccatct ggaagtgctg gatctgggcc tgaacgaaat tggccaggaa 1260
   ctgaccggcc aggaatggcg cggcctggaa aacatttttg aaatttatct gagctataac 1320
   aaatatctgc agctgacccg caacagcttt gcgctggtgc cgagcctgca gcgcctgatg 1380
   ctgcgccgcg tggcgctgaa aaacgtggat agcagcccga gcccgtttca gccgctgcgc 1440
   aacctgacca ttctggatct gagcaacaac aacattgcga acattaacga tgatatgctg 1500
   gaaggcctgg aaaaactgga aattctggat ctgcagcata acaacctggc gcgcctgtgg 1560
   aaacatgcga acccgggcgg cccgatttat tttctgaaag gcctgagcca tctgcatatt 1620
   ctgaacctgg aaagcaacgg ctttgatgaa attccggtgg aagtgtttaa agatctgttt 1680
   gaactgaaaa ttattgatct gggcctgaac aacctgaaca ccctgccggc gagcgtgttt 1740
   aacaaccagg tgagcctgaa aagcctgaac ctgcagaaaa acctgattac cagcgtggaa 1800
   aaaaaagtgt ttggcccggc gtttcgcaac ctgaccgaac tggatatgcg ctttaacccg 1860
   tttgattgca cctgcgaaag cattgcgtgg tttgtgaact ggattaacga aacccatacc 1920
   aacattccgg aactgagcag ccattatctg tgcaacaccc cgccgcatta tcatggcttt 1980
   ccggtgcgcc tgtttgatac cagcagctgc aaagatagcg cgccgtttga a          2031
<210> 8
   <211> 677
   <212> PRT
   <213> Artificial Sequence
<220>
   <223> Artificial amino acid sequence derived from the Homo sapiens hTLR3 ECD which lacks a signal peptide and contains an alanine (A) substitution mutation at N221.
<400> 8
   Lys Cys Thr Val Ser His Glu Val Ala Asp Cys Ser His Leu Lys Leu
   1               5                   10                  15
   Thr Gln Val Pro Asp Asp Leu Pro Thr Asn Ile Thr Val Leu Asn Leu
               20                  25                  30
   Thr His Asn Gln Leu Arg Arg Leu Pro Ala Ala Asn Phe Thr Arg Tyr
           35                  40                  45
   Ser Gln Leu Thr Ser Leu Asp Val Gly Phe Asn Thr Ile Ser Lys Leu
       50                  55                  60
   Glu Pro Glu Leu Cys Gln Lys Leu Pro Met Leu Lys Val Leu Asn Leu
   65                  70                  75                  80
   Gln His Asn Glu Leu Ser Gln Leu Ser Asp Lys Thr Phe Ala Phe Cys
                   85                  90                  95
   Thr Asn Leu Thr Glu Leu His Leu Met Ser Asn Ser Ile Gln Lys Ile
               100                 105                 110
   Lys Asn Asn Pro Phe Val Lys Gln Lys Asn Leu Ile Thr Leu Asp Leu
           115                 120                 125
   Ser His Asn Gly Leu Ser Ser Thr Lys Leu Gly Thr Gln Val Gln Leu
       130                 135                 140
   Glu Asn Leu Gln Glu Leu Leu Leu Ser Asn Asn Lys Ile Gln Ala Leu
   145                 150                 155                 160
   Lys Ser Glu Glu Leu Asp Ile Phe Ala Asn Ser Ser Leu Lys Lys Leu
                   165                 170                 175
   Glu Leu Ser Ser Asn Gln Ile Lys Glu Phe Ser Pro Gly Cys Phe His
               180                 185                 190
   Ala Ile Gly Arg Leu Phe Gly Leu Phe Leu Asn Asn Val Gln Leu Gly
           195                 200                 205
   Pro Ser Leu Thr Glu Lys Leu Cys Leu Glu Leu Ala Ala Thr Ser Ile
       210                 215                 220
   Arg Asn Leu Ser Leu Ser Asn Ser Gln Leu Ser Thr Thr Ser Asn Thr
   225                 230                 235                 240
   Thr Phe Leu Gly Leu Lys Trp Thr Asn Leu Thr Met Leu Asp Leu Ser
                   245                 250                 255
   Tyr Asn Asn Leu Asn Val Val Gly Asn Asp Ser Phe Ala Trp Leu Pro
               260                 265                 270
   Gln Leu Glu Tyr Phe Phe Leu Glu Tyr Asn Asn Ile Gln His Leu Phe
           275                 280                 285
   Ser His Ser Leu His Gly Leu Phe Asn Val Arg Tyr Leu Asn Leu Lys
       290                 295                 300
   Arg Ser Phe Thr Lys Gln Ser Ile Ser Leu Ala Ser Leu Pro Lys Ile
   305                 310                 315                 320
   Asp Asp Phe Ser Phe Gln Trp Leu Lys Cys Leu Glu His Leu Asn Met
                   325                 330                 335
   Glu Asp Asn Asp Ile Pro Gly Ile Lys Ser Asn Met Phe Thr Gly Leu
               340                 345                 350
   Ile Asn Leu Lys Tyr Leu Ser Leu Ser Asn Ser Phe Thr Ser Leu Arg
           355                 360                 365
   Thr Leu Thr Asn Glu Thr Phe Val Ser Leu Ala His Ser Pro Leu His
       370                 375                 380
   Ile Leu Asn Leu Thr Lys Asn Lys Ile Ser Lys Ile Glu Ser Asp Ala
   385                 390                 395                 400
   Phe Ser Trp Leu Gly His Leu Glu Val Leu Asp Leu Gly Leu Asn Glu
                   405                 410                 415
   Ile Gly Gln Glu Leu Thr Gly Gln Glu Trp Arg Gly Leu Glu Asn Ile
               420                 425                 430
   Phe Glu Ile Tyr Leu Ser Tyr Asn Lys Tyr Leu Gln Leu Thr Arg Asn
           435                 440                 445
   Ser Phe Ala Leu Val Pro Ser Leu Gln Arg Leu Met Leu Arg Arg Val
       450                 455                 460
   Ala Leu Lys Asn Val Asp Ser Ser Pro Ser Pro Phe Gln Pro Leu Arg
   465                 470                 475                 480
   Asn Leu Thr Ile Leu Asp Leu Ser Asn Asn Asn Ile Ala Asn Ile Asn
                   485                 490                 495
   Asp Asp Met Leu Glu Gly Leu Glu Lys Leu Glu Ile Leu Asp Leu Gln
               500                 505                 510
   His Asn Asn Leu Ala Arg Leu Trp Lys His Ala Asn Pro Gly Gly Pro
           515                 520                 525
   Ile Tyr Phe Leu Lys Gly Leu Ser His Leu His Ile Leu Asn Leu Glu
       530                 535                 540
   Ser Asn Gly Phe Asp Glu Ile Pro Val Glu Val Phe Lys Asp Leu Phe
   545                 550                 555                 560
   Glu Leu Lys Ile Ile Asp Leu Gly Leu Asn Asn Leu Asn Thr Leu Pro
                   565                 570                 575
   Ala Ser Val Phe Asn Asn Gln Val Ser Leu Lys Ser Leu Asn Leu Gln
               580                 585                 590
   Lys Asn Leu Ile Thr Ser Val Glu Lys Lys Val Phe Gly Pro Ala Phe
           595                 600                 605
   Arg Asn Leu Thr Glu Leu Asp Met Arg Phe Asn Pro Phe Asp Cys Thr
       610                 615                 620
   Cys Glu Ser Ile Ala Trp Phe Val Asn Trp Ile Asn Glu Thr His Thr
   625                 630                 635                 640
   Asn Ile Pro Glu Leu Ser Ser His Tyr Leu Cys Asn Thr Pro Pro His
                   645                 650                 655
   Tyr His Gly Phe Pro Val Arg Leu Phe Asp Thr Ser Ser Cys Lys Asp
               660                 665                 670
   Ser Ala Pro Phe Glu
           675
<210> 9
   <211> 2031
   <212> DNA
   <213> Artificial Sequence
<220>
   <223> Artificial cDNA sequence derived from the Homo sapiens hTLR3 ECD which lacks a signal peptide and contains an alanine (A) substitution mutation at N387.
<400> 9
   aaatgcaccg tgagccatga agtggcggat tgcagccatc tgaaactgac ccaggtgccg 60
   gatgatctgc cgaccaacat taccgtgctg aacctgaccc ataaccagct gcgccgcctg 120
   ccggcggcga actttacccg ctatagccag ctgaccagcc tggatgtggg ctttaacacc 180
   attagcaaac tggaaccgga actgtgccag aaactgccga tgctgaaagt gctgaacctg 240
   cagcataacg aactgagcca gctgagcgat aaaacctttg cgttttgcac caacctgacc 300
   gaactgcatc tgatgagcaa cagcattcag aaaattaaaa acaacccgtt tgtgaaacag 360
   aaaaacctga ttaccctgga tctgagccat aacggcctga gcagcaccaa actgggcacc 420
   caggtgcagc tggaaaacct gcaggaactg ctgctgagca acaacaaaat tcaggcgctg 480
   aaaagcgaag aactggatat ttttgcgaac agcagcctga aaaaactgga actgagcagc 540
   aaccagatta aagaatttag cccgggctgc tttcatgcga ttggccgcct gtttggcctg 600
   tttctgaaca acgtgcagct gggcccgagc ctgaccgaaa aactgtgcct ggaactggcg 660
   aacaccagca ttcgcaacct gagcctgagc aacagccagc tgagcaccac cagcaacacc 720
   acctttctgg gcctgaaatg gaccaacctg accatgctgg atctgagcta taacaacctg 780
   aacgtggtgg gcaacgatag ctttgcgtgg ctgccgcagc tggaatattt ttttctggaa 840
   tataacaaca ttcagcatct gtttagccat agcctgcatg gcctgtttaa cgtgcgctat 900
   ctgaacctga aacgcagctt taccaaacag agcattagcc tggcgagcct gccgaaaatt 960
   gatgatttta gctttcagtg gctgaaatgc ctggaacatc tgaacatgga agataacgat 1020
   attccgggca ttaaaagcaa catgtttacc ggcctgatta acctgaaata tctgagcctg 1080
   agcaacagct ttaccagcct gcgcaccctg accaacgaaa cctttgtgag cctggcgcat 1140
   agcccgctgc atattctggc gctgaccaaa aacaaaatta gcaaaattga aagcgatgcg 1200
   tttagctggc tgggccatct ggaagtgctg gatctgggcc tgaacgaaat tggccaggaa 1260
   ctgaccggcc aggaatggcg cggcctggaa aacatttttg aaatttatct gagctataac 1320
   aaatatctgc agctgacccg caacagcttt gcgctggtgc cgagcctgca gcgcctgatg 1380
   ctgcgccgcg tggcgctgaa aaacgtggat agcagcccga gcccgtttca gccgctgcgc 1440
   aacctgacca ttctggatct gagcaacaac aacattgcga acattaacga tgatatgctg 1500
   gaaggcctgg aaaaactgga aattctggat ctgcagcata acaacctggc gcgcctgtgg 1560
   aaacatgcga acccgggcgg cccgatttat tttctgaaag gcctgagcca tctgcatatt 1620
   ctgaacctgg aaagcaacgg ctttgatgaa attccggtgg aagtgtttaa agatctgttt 1680
   gaactgaaaa ttattgatct gggcctgaac aacctgaaca ccctgccggc gagcgtgttt 1740
   aacaaccagg tgagcctgaa aagcctgaac ctgcagaaaa acctgattac cagcgtggaa 1800
   aaaaaagtgt ttggcccggc gtttcgcaac ctgaccgaac tggatatgcg ctttaacccg 1860
   tttgattgca cctgcgaaag cattgcgtgg tttgtgaact ggattaacga aacccatacc 1920
   aacattccgg aactgagcag ccattatctg tgcaacaccc cgccgcatta tcatggcttt 1980
   ccggtgcgcc tgtttgatac cagcagctgc aaagatagcg cgccgtttga a          2031
<210> 10
   <211> 677
   <212> PRT
   <213> Artificial Sequence
<220>
   <223> Artificial amino acid sequence derived from the Homo sapiens hTLR3 ECD which lacks a signal peptide and contains an alanine (A) substitution mutation at N387.
<400> 10
   Lys Cys Thr Val Ser His Glu Val Ala Asp Cys Ser His Leu Lys Leu
   1               5                   10                  15
   Thr Gln Val Pro Asp Asp Leu Pro Thr Asn Ile Thr Val Leu Asn Leu
               20                  25                  30
   Thr His Asn Gln Leu Arg Arg Leu Pro Ala Ala Asn Phe Thr Arg Tyr
           35                  40                  45
   Ser Gln Leu Thr Ser Leu Asp Val Gly Phe Asn Thr Ile Ser Lys Leu
       50                  55                  60
   Glu Pro Glu Leu Cys Gln Lys Leu Pro Met Leu Lys Val Leu Asn Leu
   65                  70                  75                  80
   Gln His Asn Glu Leu Ser Gln Leu Ser Asp Lys Thr Phe Ala Phe Cys
                   85                  90                  95
   Thr Asn Leu Thr Glu Leu His Leu Met Ser Asn Ser Ile Gln Lys Ile
               100                 105                 110
   Lys Asn Asn Pro Phe Val Lys Gln Lys Asn Leu Ile Thr Leu Asp Leu
           115                 120                 125
   Ser His Asn Gly Leu Ser Ser Thr Lys Leu Gly Thr Gln Val Gln Leu
       130                 135                 140
   Glu Asn Leu Gln Glu Leu Leu Leu Ser Asn Asn Lys Ile Gln Ala Leu
   145                 150                 155                 160
   Lys Ser Glu Glu Leu Asp Ile Phe Ala Asn Ser Ser Leu Lys Lys Leu
                   165                 170                 175
   Glu Leu Ser Ser Asn Gln Ile Lys Glu Phe Ser Pro Gly Cys Phe His
               180                 185                 190
   Ala Ile Gly Arg Leu Phe Gly Leu Phe Leu Asn Asn Val Gln Leu Gly
           195                 200                 205
   Pro Ser Leu Thr Glu Lys Leu Cys Leu Glu Leu Ala Asn Thr Ser Ile
       210                 215                 220
   Arg Asn Leu Ser Leu Ser Asn Ser Gln Leu Ser Thr Thr Ser Asn Thr
   225                 230                 235                 240
   Thr Phe Leu Gly Leu Lys Trp Thr Asn Leu Thr Met Leu Asp Leu Ser
                   245                 250                 255
   Tyr Asn Asn Leu Asn Val Val Gly Asn Asp Ser Phe Ala Trp Leu Pro
               260                 265                 270
   Gln Leu Glu Tyr Phe Phe Leu Glu Tyr Asn Asn Ile Gln His Leu Phe
           275                 280                 285
   Ser His Ser Leu His Gly Leu Phe Asn Val Arg Tyr Leu Asn Leu Lys
       290                 295                 300
   Arg Ser Phe Thr Lys Gln Ser Ile Ser Leu Ala Ser Leu Pro Lys Ile
   305                 310                 315                 320
   Asp Asp Phe Ser Phe Gln Trp Leu Lys Cys Leu Glu His Leu Asn Met
                   325                 330                 335
   Glu Asp Asn Asp Ile Pro Gly Ile Lys Ser Asn Met Phe Thr Gly Leu
               340                 345                 350
   Ile Asn Leu Lys Tyr Leu Ser Leu Ser Asn Ser Phe Thr Ser Leu Arg
           355                 360                 365
   Thr Leu Thr Asn Glu Thr Phe Val Ser Leu Ala His Ser Pro Leu His
       370                 375                 380
   Ile Leu Ala Leu Thr Lys Asn Lys Ile Ser Lys Ile Glu Ser Asp Ala
   385                 390                 395                 400
   Phe Ser Trp Leu Gly His Leu Glu Val Leu Asp Leu Gly Leu Asn Glu
                   405                 410                 415
   Ile Gly Gln Glu Leu Thr Gly Gln Glu Trp Arg Gly Leu Glu Asn Ile
               420                 425                 430
   Phe Glu Ile Tyr Leu Ser Tyr Asn Lys Tyr Leu Gln Leu Thr Arg Asn
           435                 440                 445
   Ser Phe Ala Leu Val Pro Ser Leu Gln Arg Leu Met Leu Arg Arg Val
       450                 455                 460
   Ala Leu Lys Asn Val Asp Ser Ser Pro Ser Pro Phe Gln Pro Leu Arg
   465                 470                 475                 480
   Asn Leu Thr Ile Leu Asp Leu Ser Asn Asn Asn Ile Ala Asn Ile Asn
                   485                 490                 495
   Asp Asp Met Leu Glu Gly Leu Glu Lys Leu Glu Ile Leu Asp Leu Gln
               500                 505                 510
   His Asn Asn Leu Ala Arg Leu Trp Lys His Ala Asn Pro Gly Gly Pro
           515                 520                 525
   Ile Tyr Phe Leu Lys Gly Leu Ser His Leu His Ile Leu Asn Leu Glu
       530                 535                 540
   Ser Asn Gly Phe Asp Glu Ile Pro Val Glu Val Phe Lys Asp Leu Phe
   545                 550                 555                 560
   Glu Leu Lys Ile Ile Asp Leu Gly Leu Asn Asn Leu Asn Thr Leu Pro
                   565                 570                 575
   Ala Ser Val Phe Asn Asn Gln Val Ser Leu Lys Ser Leu Asn Leu Gln
               580                 585                 590
   Lys Asn Leu Ile Thr Ser Val Glu Lys Lys Val Phe Gly Pro Ala Phe
           595                 600                 605
   Arg Asn Leu Thr Glu Leu Asp Met Arg Phe Asn Pro Phe Asp Cys Thr
       610                 615                 620
   Cys Glu Ser Ile Ala Trp Phe Val Asn Trp Ile Asn Glu Thr His Thr
   625                 630                 635                 640
   Asn Ile Pro Glu Leu Ser Ser His Tyr Leu Cys Asn Thr Pro Pro His
                   645                 650                 655
   Tyr His Gly Phe Pro Val Arg Leu Phe Asp Thr Ser Ser Cys Lys Asp
               660                 665                 670
   Ser Ala Pro Phe Glu
           675
<210> 11
   <211> 2031
   <212> DNA
   <213> Artificial Sequence
<220>
   <223> Artificial cDNA sequence derived from the Homo sapiens hTLR3 ECD which lacks a signal peptide and contains alanine (A) substitution mutations at N221 and N387.
<400> 11
   aaatgcaccg tgagccatga agtggcggat tgcagccatc tgaaactgac ccaggtgccg 60
   gatgatctgc cgaccaacat taccgtgctg aacctgaccc ataaccagct gcgccgcctg 120
   ccggcggcga actttacccg ctatagccag ctgaccagcc tggatgtggg ctttaacacc 180
   attagcaaac tggaaccgga actgtgccag aaactgccga tgctgaaagt gctgaacctg 240
   cagcataacg aactgagcca gctgagcgat aaaacctttg cgttttgcac caacctgacc 300
   gaactgcatc tgatgagcaa cagcattcag aaaattaaaa acaacccgtt tgtgaaacag 360
   aaaaacctga ttaccctgga tctgagccat aacggcctga gcagcaccaa actgggcacc 420
   caggtgcagc tggaaaacct gcaggaactg ctgctgagca acaacaaaat tcaggcgctg 480
   aaaagcgaag aactggatat ttttgcgaac agcagcctga aaaaactgga actgagcagc 540
   aaccagatta aagaatttag cccgggctgc tttcatgcga ttggccgcct gtttggcctg 600
   tttctgaaca acgtgcagct gggcccgagc ctgaccgaaa aactgtgcct ggaactggcg 660
   gcgaccagca ttcgcaacct gagcctgagc aacagccagc tgagcaccac cagcaacacc 720
   acctttctgg gcctgaaatg gaccaacctg accatgctgg atctgagcta taacaacctg 780
   aacgtggtgg gcaacgatag ctttgcgtgg ctgccgcagc tggaatattt ttttctggaa 840
   tataacaaca ttcagcatct gtttagccat agcctgcatg gcctgtttaa cgtgcgctat 900
   ctgaacctga aacgcagctt taccaaacag agcattagcc tggcgagcct gccgaaaatt 960
   gatgatttta gctttcagtg gctgaaatgc ctggaacatc tgaacatgga agataacgat 1020
   attccgggca ttaaaagcaa catgtttacc ggcctgatta acctgaaata tctgagcctg 1080
   agcaacagct ttaccagcct gcgcaccctg accaacgaaa cctttgtgag cctggcgcat 1140
   agcccgctgc atattctggc gctgaccaaa aacaaaatta gcaaaattga aagcgatgcg 1200
   tttagctggc tgggccatct ggaagtgctg gatctgggcc tgaacgaaat tggccaggaa 1260
   ctgaccggcc aggaatggcg cggcctggaa aacatttttg aaatttatct gagctataac 1320
   aaatatctgc agctgacccg caacagcttt gcgctggtgc cgagcctgca gcgcctgatg 1380
   ctgcgccgcg tggcgctgaa aaacgtggat agcagcccga gcccgtttca gccgctgcgc 1440
   aacctgacca ttctggatct gagcaacaac aacattgcga acattaacga tgatatgctg 1500
   gaaggcctgg aaaaactgga aattctggat ctgcagcata acaacctggc gcgcctgtgg 1560
   aaacatgcga acccgggcgg cccgatttat tttctgaaag gcctgagcca tctgcatatt 1620
   ctgaacctgg aaagcaacgg ctttgatgaa attccggtgg aagtgtttaa agatctgttt 1680
   gaactgaaaa ttattgatct gggcctgaac aacctgaaca ccctgccggc gagcgtgttt 1740
   aacaaccagg tgagcctgaa aagcctgaac ctgcagaaaa acctgattac cagcgtggaa 1800
   aaaaaagtgt ttggcccggc gtttcgcaac ctgaccgaac tggatatgcg ctttaacccg 1860
   tttgattgca cctgcgaaag cattgcgtgg tttgtgaact ggattaacga aacccatacc 1920
   aacattccgg aactgagcag ccattatctg tgcaacaccc cgccgcatta tcatggcttt 1980
   ccggtgcgcc tgtttgatac cagcagctgc aaagatagcg cgccgtttga a          2031
<210> 12
   <211> 677
   <212> PRT
   <213> Artificial Sequence
<220>
   <223> Artificial amino acid sequence derived from the Homo sapiens hTLR3 ECD which lacks a signal peptide and contains alanine (A) substitution mutations at N221 and N387.
<400> 12
   Lys Cys Thr Val Ser His Glu Val Ala Asp Cys Ser His Leu Lys Leu
    1               5                  10                  15
   Thr Gln Val Pro Asp Asp Leu Pro Thr Asn Ile Thr Val Leu Asn Leu
               20                  25                  30
   Thr His Asn Gln Leu Arg Arg Leu Pro Ala Ala Asn Phe Thr Arg Tyr
           35                  40                  45
   Ser Gln Leu Thr Ser Leu Asp Val Gly Phe Asn Thr Ile Ser Lys Leu
       50                  55                  60
   Glu Pro Glu Leu Cys Gln Lys Leu Pro Met Leu Lys Val Leu Asn Leu
   65                  70                  75                  80
   Gln His Asn Glu Leu Ser Gln Leu Ser Asp Lys Thr Phe Ala Phe Cys
                   85                  90                  95
   Thr Asn Leu Thr Glu Leu His Leu Met Ser Asn Ser Ile Gln Lys Ile
               100                 105                 110
   Lys Asn Asn Pro Phe Val Lys Gln Lys Asn Leu Ile Thr Leu Asp Leu
           115                 120                 125
   Ser His Asn Gly Leu Ser Ser Thr Lys Leu Gly Thr Gln Val Gln Leu
       130                 135                 140
   Glu Asn Leu Gln Glu Leu Leu Leu Ser Asn Asn Lys Ile Gln Ala Leu
   145                 150                 155                 160
   Lys Ser Glu Glu Leu Asp Ile Phe Ala Asn Ser Ser Leu Lys Lys Leu
                   165                 170                 175
   Glu Leu Ser Ser Asn Gln Ile Lys Glu Phe Ser Pro Gly Cys Phe His
               180                 185                 190
   Ala Ile Gly Arg Leu Phe Gly Leu Phe Leu Asn Asn Val Gln Leu Gly
           195                 200                 205
   Pro Ser Leu Thr Glu Lys Leu Cys Leu Glu Leu Ala Ala Thr Ser Ile
       210                 215                 220
   Arg Asn Leu Ser Leu Ser Asn Ser Gln Leu Ser Thr Thr Ser Asn Thr
   225                 230                 235                 240
   Thr Phe Leu Gly Leu Lys Trp Thr Asn Leu Thr Met Leu Asp Leu Ser
                   245                 250                 255
   Tyr Asn Asn Leu Asn Val Val Gly Asn Asp Ser Phe Ala Trp Leu Pro
               260                 265                 270
   Gln Leu Glu Tyr Phe Phe Leu Glu Tyr Asn Asn Ile Gln His Leu Phe
           275                 280                 285
   Ser His Ser Leu His Gly Leu Phe Asn Val Arg Tyr Leu Asn Leu Lys
       290                 295                 300
   Arg Ser Phe Thr Lys Gln Ser Ile Ser Leu Ala Ser Leu Pro Lys Ile
   305                 310                 315                 320
   Asp Asp Phe Ser Phe Gln Trp Leu Lys Cys Leu Glu His Leu Asn Met
                   325                 330                 335
   Glu Asp Asn Asp Ile Pro Gly Ile Lys Ser Asn Met Phe Thr Gly Leu
               340                 345                 350
   Ile Asn Leu Lys Tyr Leu Ser Leu Ser Asn Ser Phe Thr Ser Leu Arg
           355                 360                 365
   Thr Leu Thr Asn Glu Thr Phe Val Ser Leu Ala His Ser Pro Leu His
       370                 375                 380
   Ile Leu Ala Leu Thr Lys Asn Lys Ile Ser Lys Ile Glu Ser Asp Ala
   385                 390                 395                 400
   Phe Ser Trp Leu Gly His Leu Glu Val Leu Asp Leu Gly Leu Asn Glu
                   405                 410                 415
   Ile Gly Gln Glu Leu Thr Gly Gln Glu Trp Arg Gly Leu Glu Asn Ile
               420                 425                 430
   Phe Glu Ile Tyr Leu Ser Tyr Asn Lys Tyr Leu Gln Leu Thr Arg Asn
           435                 440                 445
   Ser Phe Ala Leu Val Pro Ser Leu Gln Arg Leu Met Leu Arg Arg Val
       450                 455                 460
   Ala Leu Lys Asn Val Asp Ser Ser Pro Ser Pro Phe Gln Pro Leu Arg
   465                 470                 475                 480
   Asn Leu Thr Ile Leu Asp Leu Ser Asn Asn Asn Ile Ala Asn Ile Asn
                   485                 490                 495
   Asp Asp Met Leu Glu Gly Leu Glu Lys Leu Glu Ile Leu Asp Leu Gln
               500                 505                 510
   His Asn Asn Leu Ala Arg Leu Trp Lys His Ala Asn Pro Gly Gly Pro
           515                 520                 525
   Ile Tyr Phe Leu Lys Gly Leu Ser His Leu His Ile Leu Asn Leu Glu
       530                 535                 540
   Ser Asn Gly Phe Asp Glu Ile Pro Val Glu Val Phe Lys Asp Leu Phe
   545                 550                 555                 560
   Glu Leu Lys Ile Ile Asp Leu Gly Leu Asn Asn Leu Asn Thr Leu Pro
                   565                 570                 575
   Ala Ser Val Phe Asn Asn Gln Val Ser Leu Lys Ser Leu Asn Leu Gln
               580                 585                 590
   Lys Asn Leu Ile Thr Ser Val Glu Lys Lys Val Phe Gly Pro Ala Phe
           595                 600                 605
   Arg Asn Leu Thr Glu Leu Asp Met Arg Phe Asn Pro Phe Asp Cys Thr
       610                 615                 620
   Cys Glu Ser Ile Ala Trp Phe Val Asn Trp Ile Asn Glu Thr His Thr
   625                 630                 635                 640
   Asn Ile Pro Glu Leu Ser Ser His Tyr Leu Cys Asn Thr Pro Pro His
                   645                 650                 655
   Tyr His Gly Phe Pro Val Arg Leu Phe Asp Thr Ser Ser Cys Lys Asp
               660                 665                 670
   Ser Ala Pro Phe Glu
           675
<210> 13
   <211> 2031
   <212> DNA
   <213> Artificial Sequence
<220>
   <223> Artificial cDNA sequence derived from the Mus musculus TLR3 ECD which lacks a signal peptide and contains alanine (A) substitution mutations at N221, N226, N387, and N636.
<400> 13
   cagtgcaccg tgcgctataa cgtggcggat tgcagccatc tgaaactgac ccatattccg 60
   gatgatctgc cgagcaacat taccgtgctg aacctgaccc ataaccagct gcgccgcctg 120
   ccgccgacca actttacccg ctatagccag ctggcgattc tggatgcggg ctttaacagc 180
   attagcaaac tggaaccgga actgtgccag attctgccgc tgctgaaagt gctgaacctg 240
   cagcataacg aactgagcca gattagcgat cagacctttg tgttttgcac caacctgacc 300
   gaactggatc tgatgagcaa cagcattcat aaaattaaaa gcaacccgtt taaaaaccag 360
   aaaaacctga ttaaactgga tctgagccat aacggcctga gcagcaccaa actgggcacc 420
   ggcgtgcagc tggaaaacct gcaggaactg ctgctggcga aaaacaaaat tctggcgctg 480
   cgcagcgaag aactggaatt tctgggcaac agcagcctgc gcaaactgga tctgagcagc 540
   aacccgctga aagaatttag cccgggctgc tttcagacca ttggcaaact gtttgcgctg 600
   ctgctgaaca acgcgcagct gaacccgcat ctgaccgaaa aactgtgctg ggaactgagc 660
   gcgaccagca ttcaggcgct gagcctggcg aacaaccagc tgctggcgac cagcgaaagc 720
   acctttagcg gcctgaaatg gaccaacctg acccagctgg atctgagcta taacaacctg 780
   catgatgtgg gcaacggcag ctttagctat ctgccgagcc tgcgctatct gagcctggaa 840
   tataacaaca ttcagcgcct gagcccgcgc agcttttatg gcctgagcaa cctgcgctat 900
   ctgagcctga aacgcgcgtt taccaaacag agcgtgagcc tggcgagcca tccgaacatt 960
   gatgatttta gctttcagtg gctgaaatat ctggaatatc tgaacatgga tgataacaac 1020
   attccgagca ccaaaagcaa cacctttacc ggcctggtga gcctgaaata tctgagcctg 1080
   agcaaaacct ttaccagcct gcagaccctg accaacgaaa cctttgtgag cctggcgcat 1140
   agcccgctgc tgaccctggc gctgaccaaa aaccatatta gcaaaattgc gaacggcacc 1200
   tttagctggc tgggccagct gcgcattctg gatctgggcc tgaacgaaat tgaacagaaa 1260
   ctgagcggcc aggaatggcg cggcctgcgc aacatttttg aaatttatct gagctataac 1320
   aaatatctgc agctgagcac cagcagcttt gcgctggtgc cgagcctgca gcgcctgatg 1380
   ctgcgccgcg tggcgctgaa aaacgtggat attagcccga gcccgtttcg cccgctgcgc 1440
   aacctgacca ttctggatct gagcaacaac aacattgcga acattaacga agatctgctg 1500
   gaaggcctgg aaaacctgga aattctggat tttcagcata acaacctggc gcgcctgtgg 1560
   aaacgcgcga acccgggcgg cccggtgaac tttctgaaag gcctgagcca tctgcatatt 1620
   ctgaacctgg aaagcaacgg cctggatgaa attccggtgg gcgtgtttaa aaacctgttt 1680
   gaactgaaaa gcattaacct gggcctgaac aacctgaaca aactggaacc gtttattttt 1740
   gatgatcaga ccagcctgcg cagcctgaac ctgcagaaaa acctgattac cagcgtggaa 1800
   aaagatgtgt ttggcccgcc gtttcagaac ctgaacagcc tggatatgcg ctttaacccg 1860
   tttgattgca cctgcgaaag cattagctgg tttgtgaact ggattgcgca gacccatacc 1920
   aacattagcg aactgagcac ccattatctg tgcaacaccc cgcatcatta ttatggcttt 1980
   ccgctgaaac tgtttgatac cagcagctgc aaagatagcg cgccgtttga a          2031
<210> 14
   <211> 677
   <212> PRT
   <213> Artificial Sequence
<220>
   <223> Artificial amino acid sequence derived from the Mus musculus TLR3 ECD which lacks a signal peptide and contains alanine (A) substitution mutations at N221, N226, N387, and N636.
<400> 14
   Gln Cys Thr Val Arg Tyr Asn Val Ala Asp Cys Ser His Leu Lys Leu
    1               5                  10                  15
   Thr His Ile Pro Asp Asp Leu Pro Ser Asn Ile Thr Val Leu Asn Leu
               20                  25                  30
   Thr His Asn Gln Leu Arg Arg Leu Pro Pro Thr Asn Phe Thr Arg Tyr
           35                  40                  45
   Ser Gln Leu Ala Ile Leu Asp Ala Gly Phe Asn Ser Ile Ser Lys Leu
       50                  55                  60
   Glu Pro Glu Leu Cys Gln Ile Leu Pro Leu Leu Lys Val Leu Asn Leu
   65                  70                  75                  80
   Gln His Asn Glu Leu Ser Gln Ile Ser Asp Gln Thr Phe Val Phe Cys
                   85                  90                  95
   Thr Asn Leu Thr Glu Leu Asp Leu Met Ser Asn Ser Ile His Lys Ile
               100                 105                 110
   Lys Ser Asn Pro Phe Lys Asn Gln Lys Asn Leu Ile Lys Leu Asp Leu
           115                 120                 125
   Ser His Asn Gly Leu Ser Ser Thr Lys Leu Gly Thr Gly Val Gln Leu
       130                 135                 140
   Glu Asn Leu Gln Glu Leu Leu Leu Ala Lys Asn Lys Ile Leu Ala Leu
   145                 150                 155                 160
   Arg Ser Glu Glu Leu Glu Phe Leu Gly Asn Ser Ser Leu Arg Lys Leu
                   165                 170                 175
   Asp Leu Ser Ser Asn Pro Leu Lys Glu Phe Ser Pro Gly Cys Phe Gln
               180                 185                 190
   Thr Ile Gly Lys Leu Phe Ala Leu Leu Leu Asn Asn Ala Gln Leu Asn
           195                 200                 205
   Pro His Leu Thr Glu Lys Leu Cys Trp Glu Leu Ser Ala Thr Ser Ile
       210                 215                 220
   Gln Ala Leu Ser Leu Ala Asn Asn Gln Leu Leu Ala Thr Ser Glu Ser
   225                 230                 235                 240
   Thr Phe Ser Gly Leu Lys Trp Thr Asn Leu Thr Gln Leu Asp Leu Ser
                   245                 250                 255
   Tyr Asn Asn Leu His Asp Val Gly Asn Gly Ser Phe Ser Tyr Leu Pro
               260                 265                 270
   Ser Leu Arg Tyr Leu Ser Leu Glu Tyr Asn Asn Ile Gln Arg Leu Ser
           275                 280                 285
   Pro Arg Ser Phe Tyr Gly Leu Ser Asn Leu Arg Tyr Leu Ser Leu Lys
       290                 295                 300
   Arg Ala Phe Thr Lys Gln Ser Val Ser Leu Ala Ser His Pro Asn Ile
   305                 310                 315                 320
   Asp Asp Phe Ser Phe Gln Trp Leu Lys Tyr Leu Glu Tyr Leu Asn Met
                   325                 330                 335
   Asp Asp Asn Asn Ile Pro Ser Thr Lys Ser Asn Thr Phe Thr Gly Leu
               340                 345                 350
   Val Ser Leu Lys Tyr Leu Ser Leu Ser Lys Thr Phe Thr Ser Leu Gln
           355                 360                 365
   Thr Leu Thr Asn Glu Thr Phe Val Ser Leu Ala His Ser Pro Leu Leu
       370                 375                 380
   Thr Leu Ala Leu Thr Lys Asn His Ile Ser Lys Ile Ala Asn Gly Thr
   385                 390                 395                 400
   Phe Ser Trp Leu Gly Gln Leu Arg Ile Leu Asp Leu Gly Leu Asn Glu
                   405                 410                 415
   Ile Glu Gln Lys Leu Ser Gly Gln Glu Trp Arg Gly Leu Arg Asn Ile
               420                 425                 430
   Phe Glu Ile Tyr Leu Ser Tyr Asn Lys Tyr Leu Gln Leu Ser Thr Ser
           435                 440                 445
   Ser Phe Ala Leu Val Pro Ser Leu Gln Arg Leu Met Leu Arg Arg Val
       450                 455                 460
   Ala Leu Lys Asn Val Asp Ile Ser Pro Ser Pro Phe Arg Pro Leu Arg
   465                 470                 475                 480
   Asn Leu Thr Ile Leu Asp Leu Ser Asn Asn Asn Ile Ala Asn Ile Asn
                   485                 490                 495
   Glu Asp Leu Leu Glu Gly Leu Glu Asn Leu Glu Ile Leu Asp Phe Gln
               500                 505                 510
   His Asn Asn Leu Ala Arg Leu Trp Lys Arg Ala Asn Pro Gly Gly Pro
           515                 520                 525
   Val Asn Phe Leu Lys Gly Leu Ser His Leu His Ile Leu Asn Leu Glu
       530                 535                 540
   Ser Asn Gly Leu Asp Glu Ile Pro Val Gly Val Phe Lys Asn Leu Phe
   545                 550                 555                 560
   Glu Leu Lys Ser Ile Asn Leu Gly Leu Asn Asn Leu Asn Lys Leu Glu
                   565                 570                 575
   Pro Phe Ile Phe Asp Asp Gln Thr Ser Leu Arg Ser Leu Asn Leu Gln
               580                 585                 590
   Lys Asn Leu Ile Thr Ser Val Glu Lys Asp Val Phe Gly Pro Pro Phe
           595                 600                 605
   Gln Asn Leu Asn Ser Leu Asp Met Arg Phe Asn Pro Phe Asp Cys Thr
       610                 615                 620
   Cys Glu Ser Ile Ser Trp Phe Val Asn Trp Ile Ala Gln Thr His Thr
   625                 630                 635                 640
   Asn Ile Ser Glu Leu Ser Thr His Tyr Leu Cys Asn Thr Pro His His
                   645                 650                 655
   Tyr Tyr Gly Phe Pro Leu Lys Leu Phe Asp Thr Ser Ser Cys Lys Asp
               660                 665                 670
   Ser Ala Pro Phe Glu
           675
<210> 15
   <211> 2031
   <212> DNA
   <213> Artificial Sequence
<220>
   <223> Artificial cDNA sequence derived from the Homo sapiens hTLR3 ECD which lacks a signal peptide and contains an alanine (A) substitution mutation at N226.
<400> 15
   aaatgcaccg tgagccatga agtggcggat tgcagccatc tgaaactgac ccaggtgccg 60
   gatgatctgc cgaccaacat taccgtgctg aacctgaccc ataaccagct gcgccgcctg 120
   ccggcggcga actttacccg ctatagccag ctgaccagcc tggatgtggg ctttaacacc 180
   attagcaaac tggaaccgga actgtgccag aaactgccga tgctgaaagt gctgaacctg 240
   cagcataacg aactgagcca gctgagcgat aaaacctttg cgttttgcac caacctgacc 300
   gaactgcatc tgatgagcaa cagcattcag aaaattaaaa acaacccgtt tgtgaaacag 360
   aaaaacctga ttaccctgga tctgagccat aacggcctga gcagcaccaa actgggcacc 420
   caggtgcagc tggaaaacct gcaggaactg ctgctgagca acaacaaaat tcaggcgctg 480
   aaaagcgaag aactggatat ttttgcgaac agcagcctga aaaaactgga actgagcagc 540
   aaccagatta aagaatttag cccgggctgc tttcatgcga ttggccgcct gtttggcctg 600
   tttctgaaca acgtgcagct gggcccgagc ctgaccgaaa aactgtgcct ggaactggcg 660
   aacaccagca ttcgcgcgct gagcctgagc aacagccagc tgagcaccac cagcaacacc 720
   acctttctgg gcctgaaatg gaccaacctg accatgctgg atctgagcta taacaacctg 780
   aacgtggtgg gcaacgatag ctttgcgtgg ctgccgcagc tggaatattt ttttctggaa 840
   tataacaaca ttcagcatct gtttagccat agcctgcatg gcctgtttaa cgtgcgctat 900
   ctgaacctga aacgcagctt taccaaacag agcattagcc tggcgagcct gccgaaaatt 960
   gatgatttta gctttcagtg gctgaaatgc ctggaacatc tgaacatgga agataacgat 1020
   attccgggca ttaaaagcaa catgtttacc ggcctgatta acctgaaata tctgagcctg 1080
   agcaacagct ttaccagcct gcgcaccctg accaacgaaa cctttgtgag cctggcgcat 1140
   agcccgctgc atattctgaa cctgaccaaa aacaaaatta gcaaaattga aagcgatgcg 1200
   tttagctggc tgggccatct ggaagtgctg gatctgggcc tgaacgaaat tggccaggaa 1260
   ctgaccggcc aggaatggcg cggcctggaa aacatttttg aaatttatct gagctataac 1320
   aaatatctgc agctgacccg caacagcttt gcgctggtgc cgagcctgca gcgcctgatg 1380
   ctgcgccgcg tggcgctgaa aaacgtggat agcagcccga gcccgtttca gccgctgcgc 1440
   aacctgacca ttctggatct gagcaacaac aacattgcga acattaacga tgatatgctg 1500
   gaaggcctgg aaaaactgga aattctggat ctgcagcata acaacctggc gcgcctgtgg 1560
   aaacatgcga acccgggcgg cccgatttat tttctgaaag gcctgagcca tctgcatatt 1620
   ctgaacctgg aaagcaacgg ctttgatgaa attccggtgg aagtgtttaa agatctgttt 1680
   gaactgaaaa ttattgatct gggcctgaac aacctgaaca ccctgccggc gagcgtgttt 1740
   aacaaccagg tgagcctgaa aagcctgaac ctgcagaaaa acctgattac cagcgtggaa 1800
   aaaaaagtgt ttggcccggc gtttcgcaac ctgaccgaac tggatatgcg ctttaacccg 1860
   tttgattgca cctgcgaaag cattgcgtgg tttgtgaact ggattaacga aacccatacc 1920
   aacattccgg aactgagcag ccattatctg tgcaacaccc cgccgcatta tcatggcttt 1980
   ccggtgcgcc tgtttgatac cagcagctgc aaagatagcg cgccgtttga a          2031
<210> 16
   <211> 677
   <212> PRT
   <213> Artificial Sequence
<220>
   <223> Artificial amino acid sequence derived from the Homo sapiens hTLR3 ECD which lacks a signal peptide and contains an alanine (A) substitution mutation at N226.
<400> 16
   Lys Cys Thr Val Ser His Glu Val Ala Asp Cys Ser His Leu Lys Leu
   1               5                   10                  15
   Thr Gln Val Pro Asp Asp Leu Pro Thr Asn Ile Thr Val Leu Asn Leu
               20                  25                  30
   Thr His Asn Gln Leu Arg Arg Leu Pro Ala Ala Asn Phe Thr Arg Tyr
           35                  40                  45
   Ser Gln Leu Thr Ser Leu Asp Val Gly Phe Asn Thr Ile Ser Lys Leu
       50                  55                  60
   Glu Pro Glu Leu Cys Gln Lys Leu Pro Met Leu Lys Val Leu Asn Leu
   65                  70                  75                  80
   Gln His Asn Glu Leu Ser Gln Leu Ser Asp Lys Thr Phe Ala Phe Cys
                   85                  90                  95
   Thr Asn Leu Thr Glu Leu His Leu Met Ser Asn Ser Ile Gln Lys Ile
   100 105 110
   Lys Asn Asn Pro Phe Val Lys Gln Lys Asn Leu Ile Thr Leu Asp Leu
   115 120 125
   Ser His Asn Gly Leu Ser Ser Thr Lys Leu Gly Thr Gln Val Gln Leu
   130 135 140
   Glu Asn Leu Gln Glu Leu Leu Leu Ser Asn Asn Lys Ile Gln Ala Leu
   145 150 155 160
   Lys Ser Glu Glu Leu Asp Ile Phe Ala Asn Ser Ser Leu Lys Lys Leu
   165 170 175
   Glu Leu Ser Ser Asn Gln Ile Lys Glu Phe Ser Pro Gly Cys Phe His
   180 185 190
   Ala Ile Gly Arg Leu Phe Gly Leu Phe Leu Asn Asn Val Gln Leu Gly
   195 200 205
   Pro Ser Leu Thr Glu Lys Leu Cys Leu Glu Leu Ala Asn Thr Ser Ile
   210 215 220
   Arg Ala Leu Ser Leu Ser Asn Ser Gln Leu Ser Thr Thr Ser Asn Thr
   225 230 235 240
   Thr Phe Leu Gly Leu Lys Trp Thr Asn Leu Thr Met Leu Asp Leu Ser
   245 250 255
   Tyr Asn Asn Leu Asn Val Val Gly Asn Asp Ser Phe Ala Trp Leu Pro
   260 265 270
   Gln Leu Glu Tyr Phe Phe Leu Glu Tyr Asn Asn Ile Gln His Leu Phe
   275 280 285
   Ser His Ser Leu His Gly Leu Phe Asn Val Arg Tyr Leu Asn Leu Lys
   290 295 300
   Arg Ser Phe Thr Lys Gln Ser Ile Ser Leu Ala Ser Leu Pro Lys Ile
   305 310 315 320
   Asp Asp Phe Ser Phe Gln Trp Leu Lys Cys Leu Glu His Leu Asn Met
   325 330 335
   Glu Asp Asn Asp Ile Pro Gly Ile Lys Ser Asn Met Phe Thr Gly Leu
   340 345 350
   Ile Asn Leu Lys Tyr Leu Ser Leu Ser Asn Ser Phe Thr Ser Leu Arg
   355 360 365
   Thr Leu Thr Asn Glu Thr Phe Val Ser Leu Ala His Ser Pro Leu His
   370 375 380
   Ile Leu Asn Leu Thr Lys Asn Lys Ile Ser Lys Ile Glu Ser Asp Ala
   385 390 395 400
   Phe Ser Trp Leu Gly His Leu Glu Val Leu Asp Leu Gly Leu Asn Glu
   405 410 415
   Ile Gly Gln Glu Leu Thr Gly Gln Glu Trp Arg Gly Leu Glu Asn Ile
   420 425 430
   Phe Glu Ile Tyr Leu Ser Tyr Asn Lys Tyr Leu Gln Leu Thr Arg Asn
   435 440 445
   Ser Phe Ala Leu Val Pro Ser Leu Gln Arg Leu Met Leu Arg Arg Val
   450 455 460
   Ala Leu Lys Asn Val Asp Ser Ser Pro Ser Pro Phe Gln Pro Leu Arg
   465 470 475 480
   Asn Leu Thr Ile Leu Asp Leu Ser Asn Asn Asn Ile Ala Asn Ile Asn
   485 490 495
   Asp Asp Met Leu Glu Gly Leu Glu Lys Leu Glu Ile Leu Asp Leu Gln
   500 505 510
   His Asn Asn Leu Ala Arg Leu Trp Lys His Ala Asn Pro Gly Gly Pro
   515 520 525
   Ile Tyr Phe Leu Lys Gly Leu Ser His Leu His Ile Leu Asn Leu Glu
   530 535 540
   Ser Asn Gly Phe Asp Glu Ile Pro Val Glu Val Phe Lys Asp Leu Phe
   545                 550                 555                 560
   Glu Leu Lys Ile Ile Asp Leu Gly Leu Asn Asn Leu Asn Thr Leu Pro
                   565                 570                 575
   Ala Ser Val Phe Asn Asn Gln Val Ser Leu Lys Ser Leu Asn Leu Gln
               580                 585                 590
   Lys Asn Leu Ile Thr Ser Val Glu Lys Lys Val Phe Gly Pro Ala Phe
           595                 600                 605
   Arg Asn Leu Thr Glu Leu Asp Met Arg Phe Asn Pro Phe Asp Cys Thr
       610                 615                 620
   Cys Glu Ser Ile Ala Trp Phe Val Asn Trp Ile Asn Glu Thr His Thr
   625                 630                 635                 640
   Asn Ile Pro Glu Leu Ser Ser His Tyr Leu Cys Asn Thr Pro Pro His
                   645                 650                 655
   Tyr His Gly Phe Pro Val Arg Leu Phe Asp Thr Ser Ser Cys Lys Asp
               660                 665                 670
   Ser Ala Pro Phe Glu
           675
<210> 17
   <211> 2031
   <212> DNA
   <213> Artificial Sequence
<220>
   <223> Artificial cDNA sequence derived from the Homo sapiens hTLR3 ECD which lacks a signal peptide and contains an alanine (A) substitution mutation at N636.
<400> 17
   aaatgcaccg tgagccatga agtggcggat tgcagccatc tgaaactgac ccaggtgccg 60
   gatgatctgc cgaccaacat taccgtgctg aacctgaccc ataaccagct gcgccgcctg 120
   ccggcggcga actttacccg ctatagccag ctgaccagcc tggatgtggg ctttaacacc 180
   attagcaaac tggaaccgga actgtgccag aaactgccga tgctgaaagt gctgaacctg 240
   cagcataacg aactgagcca gctgagcgat aaaacctttg cgttttgcac caacctgacc 300
   gaactgcatc tgatgagcaa cagcattcag aaaattaaaa acaacccgtt tgtgaaacag 360
   aaaaacctga ttaccctgga tctgagccat aacggcctga gcagcaccaa actgggcacc 420
   caggtgcagc tggaaaacct gcaggaactg ctgctgagca acaacaaaat tcaggcgctg 480
   aaaagcgaag aactggatat ttttgcgaac agcagcctga aaaaactgga actgagcagc 540
   aaccagatta aagaatttag cccgggctgc tttcatgcga ttggccgcct gtttggcctg 600
   tttctgaaca acgtgcagct gggcccgagc ctgaccgaaa aactgtgcct ggaactggcg 660
   aacaccagca ttcgcaacct gagcctgagc aacagccagc tgagcaccac cagcaacacc 720
   acctttctgg gcctgaaatg gaccaacctg accatgctgg atctgagcta taacaacctg 780
   aacgtggtgg gcaacgatag ctttgcgtgg ctgccgcagc tggaatattt ttttctggaa 840
   tataacaaca ttcagcatct gtttagccat agcctgcatg gcctgtttaa cgtgcgctat 900
   ctgaacctga aacgcagctt taccaaacag agcattagcc tggcgagcct gccgaaaatt 960
   gatgatttta gctttcagtg gctgaaatgc ctggaacatc tgaacatgga agataacgat 1020
   attccgggca ttaaaagcaa catgtttacc ggcctgatta acctgaaata tctgagcctg 1080
   agcaacagct ttaccagcct gcgcaccctg accaacgaaa cctttgtgag cctggcgcat 1140
   agcccgctgc atattctgaa cctgaccaaa aacaaaatta gcaaaattga aagcgatgcg 1200
   tttagctggc tgggccatct ggaagtgctg gatctgggcc tgaacgaaat tggccaggaa 1260
   ctgaccggcc aggaatggcg cggcctggaa aacatttttg aaatttatct gagctataac 1320
   aaatatctgc agctgacccg caacagcttt gcgctggtgc cgagcctgca gcgcctgatg 1380
   ctgcgccgcg tggcgctgaa aaacgtggat agcagcccga gcccgtttca gccgctgcgc 1440
   aacctgacca ttctggatct gagcaacaac aacattgcga acattaacga tgatatgctg 1500
   gaaggcctgg aaaaactgga aattctggat ctgcagcata acaacctggc gcgcctgtgg 1560
   aaacatgcga acccgggcgg cccgatttat tttctgaaag gcctgagcca tctgcatatt 1620
   ctgaacctgg aaagcaacgg ctttgatgaa attccggtgg aagtgtttaa agatctgttt 1680
   gaactgaaaa ttattgatct gggcctgaac aacctgaaca ccctgccggc gagcgtgttt 1740
   aacaaccagg tgagcctgaa aagcctgaac ctgcagaaaa acctgattac cagcgtggaa 1800
   aaaaaagtgt ttggcccggc gtttcgcaac ctgaccgaac tggatatgcg ctttaacccg 1860
   tttgattgca cctgcgaaag cattgcgtgg tttgtgaact ggattgcgga aacccatacc 1920
   aacattccgg aactgagcag ccattatctg tgcaacaccc cgccgcatta tcatggcttt 1980
   ccggtgcgcc tgtttgatac cagcagctgc aaagatagcg cgccgtttga a          2031
<210> 18
   <211> 677
   <212> PRT
   <213> Artificial Sequence
<220>
   <223> Artificial amino acid sequence derived from the Homo sapiens hTLR3 ECD which lacks a signal peptide and contains an alanine (A) substitution mutation at N636.
<400> 18
   Lys Cys Thr Val Ser His Glu Val Ala Asp Cys Ser His Leu Lys Leu
   1               5                   10                  15
   Thr Gln Val Pro Asp Asp Leu Pro Thr Asn Ile Thr Val Leu Asn Leu
               20                  25                  30
   Thr His Asn Gln Leu Arg Arg Leu Pro Ala Ala Asn Phe Thr Arg Tyr
           35                  40                  45
   Ser Gln Leu Thr Ser Leu Asp Val Gly Phe Asn Thr Ile Ser Lys Leu
       50                  55                  60
   Glu Pro Glu Leu Cys Gln Lys Leu Pro Met Leu Lys Val Leu Asn Leu
   65                  70                  75                  80
   Gln His Asn Glu Leu Ser Gln Leu Ser Asp Lys Thr Phe Ala Phe Cys
                   85                  90                  95
   Thr Asn Leu Thr Glu Leu His Leu Met Ser Asn Ser Ile Gln Lys Ile
               100                 105                 110
   Lys Asn Asn Pro Phe Val Lys Gln Lys Asn Leu Ile Thr Leu Asp Leu
           115                 120                 125
   Ser His Asn Gly Leu Ser Ser Thr Lys Leu Gly Thr Gln Val Gln Leu
       130                 135                 140
   Glu Asn Leu Gln Glu Leu Leu Leu Ser Asn Asn Lys Ile Gln Ala Leu
   145                 150                 155                 160
   Lys Ser Glu Glu Leu Asp Ile Phe Ala Asn Ser Ser Leu Lys Lys Leu
                   165                 170                 175
   Glu Leu Ser Ser Asn Gln Ile Lys Glu Phe Ser Pro Gly Cys Phe His
               180                 185                 190
   Ala Ile Gly Arg Leu Phe Gly Leu Phe Leu Asn Asn Val Gln Leu Gly
           195                 200                 205
   Pro Ser Leu Thr Glu Lys Leu Cys Leu Glu Leu Ala Asn Thr Ser Ile
       210                 215                 220
   Arg Asn Leu Ser Leu Ser Asn Ser Gln Leu Ser Thr Thr Ser Asn Thr
   225                 230                 235                 240
   Thr Phe Leu Gly Leu Lys Trp Thr Asn Leu Thr Met Leu Asp Leu Ser
                   245                 250                 255
   Tyr Asn Asn Leu Asn Val Val Gly Asn Asp Ser Phe Ala Trp Leu Pro
               260                 265                 270
   Gln Leu Glu Tyr Phe Phe Leu Glu Tyr Asn Asn Ile Gln His Leu Phe
           275                 280                 285
   Ser His Ser Leu His Gly Leu Phe Asn Val Arg Tyr Leu Asn Leu Lys
       290                 295                 300
   Arg Ser Phe Thr Lys Gln Ser Ile Ser Leu Ala Ser Leu Pro Lys Ile
   305                 310                 315                 320
   Asp Asp Phe Ser Phe Gln Trp Leu Lys Cys Leu Glu His Leu Asn Met
                   325                 330                 335
   Glu Asp Asn Asp Ile Pro Gly Ile Lys Ser Asn Met Phe Thr Gly Leu
               340                 345                 350
   Ile Asn Leu Lys Tyr Leu Ser Leu Ser Asn Ser Phe Thr Ser Leu Arg
           355                 360                 365
   Thr Leu Thr Asn Glu Thr Phe Val Ser Leu Ala His Ser Pro Leu His
       370                 375                 380
   Ile Leu Asn Leu Thr Lys Asn Lys Ile Ser Lys Ile Glu Ser Asp Ala
   385                 390                 395                 400
   Phe Ser Trp Leu Gly His Leu Glu Val Leu Asp Leu Gly Leu Asn Glu
                   405                 410                 415
   Ile Gly Gln Glu Leu Thr Gly Gln Glu Trp Arg Gly Leu Glu Asn Ile
               420                 425                 430
   Phe Glu Ile Tyr Leu Ser Tyr Asn Lys Tyr Leu Gln Leu Thr Arg Asn
           435                 440                 445
   Ser Phe Ala Leu Val Pro Ser Leu Gln Arg Leu Met Leu Arg Arg Val
       450                 455                 460
   Ala Leu Lys Asn Val Asp Ser Ser Pro Ser Pro Phe Gln Pro Leu Arg
   465                 470                 475                 480
   Asn Leu Thr Ile Leu Asp Leu Ser Asn Asn Asn Ile Ala Asn Ile Asn
                   485                 490                 495
   Asp Asp Met Leu Glu Gly Leu Glu Lys Leu Glu Ile Leu Asp Leu Gln
               500                 505                 510
   His Asn Asn Leu Ala Arg Leu Trp Lys His Ala Asn Pro Gly Gly Pro
           515                 520                 525
   Ile Tyr Phe Leu Lys Gly Leu Ser His Leu His Ile Leu Asn Leu Glu
       530                 535                 540
   Ser Asn Gly Phe Asp Glu Ile Pro Val Glu Val Phe Lys Asp Leu Phe
   545                 550                 555                 560
   Glu Leu Lys Ile Ile Asp Leu Gly Leu Asn Asn Leu Asn Thr Leu Pro
                   565                 570                 575
   Ala Ser Val Phe Asn Asn Gln Val Ser Leu Lys Ser Leu Asn Leu Gln
               580                 585                 590
   Lys Asn Leu Ile Thr Ser Val Glu Lys Lys Val Phe Gly Pro Ala Phe
           595                 600                 605
   Arg Asn Leu Thr Glu Leu Asp Met Arg Phe Asn Pro Phe Asp Cys Thr
       610                 615                 620
   Cys Glu Ser Ile Ala Trp Phe Val Asn Trp Ile Ala Glu Thr His Thr
   625                 630                 635                 640
   Asn Ile Pro Glu Leu Ser Ser His Tyr Leu Cys Asn Thr Pro Pro His
                   645                 650                 655
   Tyr His Gly Phe Pro Val Arg Leu Phe Asp Thr Ser Ser Cys Lys Asp
               660                 665                 670
   Ser Ala Pro Phe Glu
           675
<210> 19
   <211> 2031
   <212> DNA
   <213> Artificial Sequence
<220>
   <223> Artificial cDNA sequence derived from the Homo sapiens hTLR3 ECD which lacks a signal peptide and contains an alanine (A) substitution mutations at N221, N226, N387, and N636.
<400> 19
   aaatgcaccg tgagccatga agtggcggat tgcagccatc tgaaactgac ccaggtgccg 60
   gatgatctgc cgaccaacat taccgtgctg aacctgaccc ataaccagct gcgccgcctg 120
   ccggcggcga actttacccg ctatagccag ctgaccagcc tggatgtggg ctttaacacc 180
   attagcaaac tggaaccgga actgtgccag aaactgccga tgctgaaagt gctgaacctg 240
   cagcataacg aactgagcca gctgagcgat aaaacctttg cgttttgcac caacctgacc 300
   gaactgcatc tgatgagcaa cagcattcag aaaattaaaa acaacccgtt tgtgaaacag 360
   aaaaacctga ttaccctgga tctgagccat aacggcctga gcagcaccaa actgggcacc 420
   caggtgcagc tggaaaacct gcaggaactg ctgctgagca acaacaaaat tcaggcgctg 480
   aaaagcgaag aactggatat ttttgcgaac agcagcctga aaaaactgga actgagcagc 540
   aaccagatta aagaatttag cccgggctgc tttcatgcga ttggccgcct gtttggcctg 600
   tttctgaaca acgtgcagct gggcccgagc ctgaccgaaa aactgtgcct ggaactggcg 660
   gcgaccagca ttcgcgcgct gagcctgagc aacagccagc tgagcaccac cagcaacacc 720
   acctttctgg gcctgaaatg gaccaacctg accatgctgg atctgagcta taacaacctg 780
   aacgtggtgg gcaacgatag ctttgcgtgg ctgccgcagc tggaatattt ttttctggaa 840
   tataacaaca ttcagcatct gtttagccat agcctgcatg gcctgtttaa cgtgcgctat 900
   ctgaacctga aacgcagctt taccaaacag agcattagcc tggcgagcct gccgaaaatt 960
   gatgatttta gctttcagtg gctgaaatgc ctggaacatc tgaacatgga agataacgat 1020
   attccgggca ttaaaagcaa catgtttacc ggcctgatta acctgaaata tctgagcctg 1080
   agcaacagct ttaccagcct gcgcaccctg accaacgaaa cctttgtgag cctggcgcat 1140
   agcccgctgc atattctggc gctgaccaaa aacaaaatta gcaaaattga aagcgatgcg 1200
   tttagctggc tgggccatct ggaagtgctg gatctgggcc tgaacgaaat tggccaggaa 1260
   ctgaccggcc aggaatggcg cggcctggaa aacatttttg aaatttatct gagctataac 1320
   aaatatctgc agctgacccg caacagcttt gcgctggtgc cgagcctgca gcgcctgatg 1380
   ctgcgccgcg tggcgctgaa aaacgtggat agcagcccga gcccgtttca gccgctgcgc 1440
   aacctgacca ttctggatct gagcaacaac aacattgcga acattaacga tgatatgctg 1500
   gaaggcctgg aaaaactgga aattctggat ctgcagcata acaacctggc gcgcctgtgg 1560
   aaacatgcga acccgggcgg cccgatttat tttctgaaag gcctgagcca tctgcatatt 1620
   ctgaacctgg aaagcaacgg ctttgatgaa attccggtgg aagtgtttaa agatctgttt 1680
   gaactgaaaa ttattgatct gggcctgaac aacctgaaca ccctgccggc gagcgtgttt 1740
   aacaaccagg tgagcctgaa aagcctgaac ctgcagaaaa acctgattac cagcgtggaa 1800
   aaaaaagtgt ttggcccggc gtttcgcaac ctgaccgaac tggatatgcg ctttaacccg 1860
   tttgattgca cctgcgaaag cattgcgtgg tttgtgaact ggattgcgga aacccatacc 1920
   aacattccgg aactgagcag ccattatctg tgcaacaccc cgccgcatta tcatggcttt 1980
   ccggtgcgcc tgtttgatac cagcagctgc aaagatagcg cgccgtttga a          2031
<210> 20
   <211> 677
   <212> PRT
   <213> Artificial Sequence
<220>
   <223> Artificial amino acid sequence derived from the Homo sapiens hTLR3 ECD which lacks a signal peptide and contains an alanine (A) substitution mutations at N221, N226, N387, and N636.
<400> 20 ,

## Description

### Field of the Invention

The present invention relates to TLR3 glycosylation site muteins, nucleic acids encoding the muteins, and methods of modulating TLR3 activity in a cell.

### Background of the Invention

Pathologies associated with inflammatory conditions represent a significant challenge in health care and can be painful, debilitating and lethal. For example, sepsis and sepsis-associated conditions affect more than 750,000 people annually in the U.S. with mortality rates of 28-50%, resulting in 215,000 annual deaths (Natanson et al., Crit. Care Med. 26:1927-1931 (1998); Angus et al., Crit. Care Med. 29:1303-1310 (2001)). Other inflammatory conditions such as the inflammatory bowel diseases (IBD) Crohn's disease and ulcerative colitis affect more than 1 million people per year in the U.S. (Hanauer et al., Rev. Gastroenterol. Disord. 3:81-92 (2003)).

Inflammatory pulmonary conditions affecting lung function such as chronic obstructive pulmonary disease (COPD), asthma and lung infections also affect significant numbers of people in the U.S. COPD, for example, affects an estimated 10 million adult Americans and the prevalence is rising (Mapel et al., Manag. Care Interface 17:61-66 (2004)). Pathologies associated with these inflammatory conditions and exacerbations of these conditions have significant health and economic impacts.

Exacerbation in pulmonary diseases such as asthma and COPD is characterized by the worsening of symptoms and a decline in lung function. Viral infections are associated with exacerbations of many pulmonary diseases (Johnston, Am. J. Respir. Crit. Care Med. 152: S46-52 (1995); Bandi et al, FEMS Immunol. Med. Microbiol. 37: 69-75 (2003)) and are believed to be a major cause of exacerbations. Secretion of pro-inflammatory cytokines in the lungs following viral infection represents a crucial step in promoting the inflammatory response in various lung diseases (Gern et al., Am. J. Respir. Cell. Mol. Biol. 28:731-737 (2003); Panina-Bordignon et al., Curr. Opin. Pulm. Med. 9:104-110 (2003)).

Recognition of microbial antigens by the host immune system is mediated through innate immune receptors, whose activation represents an important step in the initiation of an inflammatory response. Toll-Like Receptors (TLR) represent a family of innate immune receptors that play a crucial role in mediating an immune response to foreign antigens. TLR3, for example, is a mammalian pattern recognition receptor that recognizes double-stranded (ds) RNA as well as the synthetic ds RNA analog poly-riboinosinicribocytidylic acid (poly I:C), (Alexopoulou et al., Nature 413: 732-238 (2001)). Moreover, TLR3 has been shown to recognize endogenous ligands such as mRNA released from necrotic cells (Kariko et al., J. Biol. Chem. 26: 12542-12550 (2004)) indicating that necrotic cell death at inflammation sites may contribute to activation of TLR3. A full-length human TLR3 amino acid sequence and encoding polynucleotide sequence is shown in SEQ ID NO: 1 and SEQ ID NO: 2, respectively.

Activation of TLR3 by the ds viral RNA analog poly(I:C) or by endogenous mRNA ligands induces secretion of pro-inflammatory cytokines and chemokines, a finding that indicates TLR3 activation modulates disease outcome during infection-associated inflammation. Thus, TLR3 ligation *in vivo* is thought to occur in the context of viral infection (Tabeta et al., Proc. Natl. Acad. Sci. USA 101:3516-3521 (2004)) or necrosis associated with inflammation (Kariko et al., J. Biol. Chem. 26: 12542-12550 (2004)). Overall, these data demonstrate that ligation of TLR3 initiates cascades of phosphorylation and transcriptional activation events that result in the production of numerous inflammatory cytokines that are thought to contribute to innate immunity (reviewed by Takeda and Akira, J. Derm. Sci. 34:73-82 (2004)). Further, these data suggest that sustained TLR3 activation can be a critical component in the modulation of infection-associated inflammatory diseases. Published data lend support to this hypothesis as shown by findings that associate over-production of pro-inflammatory cytokines with systemic inflammatory response syndrome, infection-associated acute cytokine storms (reviewed by Van Amersfoort et al., Clin. Microbiol. Rev. 16: 379-414 (2003)) and immune-mediated chronic conditions such as rheumatoid arthritis (reviewed by Miossec et al., Curr. Opin. Rheumatol. 16:218-222 (2004)) and inflammatory bowel diseases (reviewed by Ogata and Hibi, Curr. Pharm. Des. 9: 1107-1113 (2003)).

Importantly, it is becoming clear that TLR3 activity also plays a significant role in conditions such as inflammatory bowel disease symptoms, sepsis, cytokine, chemokine, and growth factor mediated lung pathologies and pulmonary inflammatory conditions resulting from increased inflammatory cell infiltration into lung tissues. However, it has been unclear what, if any, effect TLR3 glycosylation has on TLR3 activity and TLR3 activity mediated conditions.

Thus, a need exists to understand the effect of TLR3 glycosylation on TLR3 activity and exploit this information to develop compositions and methods that effectively modulate TLR3 activity.

### Brief Description of the Drawings

Fig. 1 panel (A) shows MALDI-TOF mass spectra of hTLR3 extracellular domain (ECD) and panel (B) shows MALDI-TOF mass spectra of hTLR3 ECD treated with deglycosidases.
Fig. 2 shows the effect of N-glycosylation with tunicamycin onpoly(I:C) induced activation of hTLR3 signaling in HEK293 cells.
Fig. 3 shows the effect of ECD N-glycosylation by mutagenesis of N247, N252, or N413 in hTLR3 (SEQ ID NO: 2) on activation of hTLR3 signaling in cells. Panel (A) is an alignment of possible N-glycosylation sites from selected hTLR3 homologs; panels (B) and (C) are hTLR3 activation assays performed with the indicated hTLR3 mutants at 10 µg/ml (B) or 2.5 µg/ml (C) of the hTLR3 ligand poly(I:C).

Fig. 4 shows the effect of hTLR3 ECD N-glycosylation by mutagenesis of N247, N252, or N662 of hTLR (SEQ ID NO: 2) on poly(I:C) induced activation of hTLR3 signaling in cells.

### Summary of the Invention

One aspect of the invention is a peptide chain comprising an amino acid sequence of SEQ ID NO: 6 and at least one mutation within 3 amino acid residues of a position aligning to N636 of the amino acid sequence SEQ ID NO: 6.

Another aspect of the invention is a peptide chain comprising at least one mutation within 3 amino acid residues of position N636 of the amino acid sequence SEQ ID NO: 6.

Another aspect of the invention is a peptide chain comprising the amino acid sequence SEQ ID NO: 14, SEQ ID NO: 18, or SEQ ID NO: 20.

Another aspect of the invention is a method of modulating TLR3 activity in a cell comprising decreasing TLR3 glycosylation in the cell wherein the modulation is attenuation.

### Detailed Description of the Invention

The term "homolog" means protein sequences having between 40% and 100% sequence identity to a reference sequence. Homologs of hTLR3 include peptide chains from other species that have between 40% and 100% sequence identity to a known hTLR3 sequence. The terms "TLR3 homolog" and "TLR3" are used interchangeably throughout the specification and claims.

The term "peptide chain" as used herein means a molecule comprising at least two naturally or non-naturally occurring amino acid residues linked by peptide bonds.

The term "TLR3 activity" as used herein refers to any activities occurring as a result of ligand binding to a cell surface TLR3 homolog or that are mediated, in whole or in part, by at least one TLR3 homolog peptide chain.

The compositions and methods of the invention are useful in modulating the activity of TLR3 homologs in cells both *in vitro* and *in vivo*. In particular, the compositions and methods of the invention can be used to attenuate *in vivo* TLR3 dependent signaling and biological processes associated with TLR3 activity in conditions such as inflammatory bowel disease symptoms, sepsis, cytokine, chemokine, and growth factor mediated lung pathologies and pulmonary inflammatory conditions resulting from increased inflammatory cell infiltration into lung tissues.

Described herein is a peptide chain comprising an amino acid sequence with at least 75% identity to the amino acid sequence of SEQ ID NO: 6 and at least one mutation within 3 amino acid residues of a position aligning to N221, N226, N387, or N636 of the amino acid sequence SEQ ID NO: 6. Percent identity between two peptide chains can be determined by alignment using the default settings of the BLASTP 2.2.12 [Aug-07-2005] algorithm with low complexity filtering turned off and using SEQ ID NO: 6 as the BLASTP query sequence. The amino acid sequences of SEQ ID NO: 8, SEQ ID NO: 10, SEQ ID NO: 12, SEQ ID NO: 14, SEQ ID NO: 16, SEQ ID NO: 18, and SEQ ID NO: 20 are exemplary of such peptide chains. Such peptide chains may comprise additional sequences in addition to the mutagenized mature form ECD TLR3 homolog. Such additional sequences may be, for example, signal peptides such as a native signal peptide or the native intracellular and transmembrane domains of the homolog. Those skilled in the art will recognize other such additional sequences such as affinity tag sequences or other sequences that facilitate TLR activity, function or purification.

Such peptide chains may be readily made by determining the percent identity between a TLR3 homolog and SEQ ID NO: 6 as described above, selecting a homolog with at least 75% identity to SEQ ID NO: 6, identifying a position aligning to N221, N226, N387, or N636 of SEQ ID NO: 6, and introducing at least one mutation within 3 residues of the position identified. Importantly, the core consensus glycosylation site motif is the 3 amino acid residue Asn-X-Ser/Thr motif. Such mutations may be substitutions, deletions, or insertions and can be generated using *in vitro* and *in vivo* mutagenesis techniques well know in the art. Such peptide chains may also comprise additional mutations incorporated into the TLR3 homolog that do not occur within 3 amino acid residues of a position aligning to N221, N226, N387, or N636 of the amino acid sequence SEQ ID NO: 6.

Described herein is a peptide chain comprising at least one mutation within 3 amino acid residues of position N221, N226, N387, or N636 of the amino acid sequence SEQ ID NO: 6. The amino acid sequences of SEQ ID NO: 8, SEQ ID NO: 10, SEQ ID NO: 12, SEQ ID NO: 16, SEQ ID NO: 18 and SEQ ID NO: 20 are exemplary of such peptide chains. Such mutations may be substitutions, deletions, or insertions and can be generated using *in vitro* and *in vivo* mutagenesis techniques well know in the art. Such peptide chains may also comprise additional sequences as discussed above.

Described herein is a peptide chain comprising the amino acid sequence SEQ ID NO: 8, SEQ ID NO: 10, SEQ ID NO: 12, SEQ ID NO: 14, SEQ ID NO: 16, SEQ ID NO: 18, or SEQ ID NO: 20. Such peptide chains may also comprise additional sequences as discussed above.

In one embodiment the invention provides a nucleic acid encoding a peptide chain of the invention.

Described herein is a nucleic acid comprising the nucleic acid sequence of SEQ ID NO: 7, SEQ ID NO: 9, SEQ ID NO: 11, SEQ ID NO: 13, SEQ ID NO: 15, SEQ ID NO: 17, or SEQ ID NO: 19.

The nucleic acids of the invention may be made using *in vivo* or *in vitro* techniques known by those skilled in the art. Such nucleic acids may comprise DNA and RNA. Additionally, such nucleic acids may comprise additional nucleic acid sequences or be conjugated to another class of molecules. For example, such nucleic acids may be inserted into vector nucleic acids or supplied to a cell or system to produce expression of a peptide chain encoded by the nucleic acid. Such cells or systems may be eukaryotic cells, prokaryotic cells, archael cells, or cell free *in vitro* systems such as *in vitro* coupled transcription and translation systems. Techniques for peptide chain expression, introducing nucleic acids into vectors and supplying nucleic acids to cells, and cells or systems suitable for expression of peptide chains encoded by a nucleic acid are well know to those skilled in the art.

Described herein is a method of modulating TLR3 activity in a cell comprising decreasing TLR3 glycosylation in the cell using techniques such as gene inactivation or transcript targeting (*e.g*. siRNA). TLR3 glycosylation may decreased, for example, by providing a cell with small molecules that inhibit TLR3 glycosylation such as tunicamycin, providing TLR3 homologs in which a glycosylation site has been modified so that it can no longer be glycosylated, over expressing glycolytic enzymes in the cell, and deceasing or inactivating expression of glycoslyases in the cell. Antibody molecules or antibody fragments may also be used to decrease TLR3 glycosylation and activity. Such antibody molecules or fragments may block ligand or receptor multimerization by binding to the region of cell surface TLR3 containing residue N221, N226, N387 or N636 of SEQ ID NO: 6. In the methods of the description both N-glycosylation and O-glycosylation of TLR3 homologs may be targeted to decrease TLR3 glycosylation.

Described herein is a method of modulating TLR3 activity in a cell comprising providing tunicamycin to the cell. Tunicamycin can be provided to a cell *in vitro* via the culture media (at a concentration of about 0.2 to 0.5 µg/ml or *in vivo* via intravenous injection for example. Those skilled in the art will recognize many other methods and routes by which tunicamycin can be provided to a cell *in vitro* or *in vivo.*

In another embodiment the invention provides a method of modulating TLR3 activity in a cell comprising providing a peptide chain of the invention to the cell. Peptide chains may be provided to a cell by, for example, supplying the peptide chain to the fluid or tissue surrounding a cell, introducing an exogenous nucleic acid expressing the peptide chain into the cell, microinjection of the peptide chain into the cell, or fusing a cell with a second cell or vesicle containing the peptide chain. Those skilled in the art will recognize other means by which to provide a peptide chain to a cell.

In another embodiment the invention provides a nucleic acid of the invention for use in a method of modulating TLR3 activity in a cell. The nucleic acids of the invention may be provided to a cell through the use of viral, plasmid, cellular, or vesicular vectors, microinjection, naturally occurring nucleic acid uptake or competency, conjugation to molecule capable of entering a cell or by any other technique known to those skilled in the art by which nucleic acids may be introduced into a cell.

In another embodiment of the invention the nucleic acid is provided to the cell by transfection. Transfection of nucleic acids into a cell may be accomplished by a variety of techniques such as electroporation, chemical shock, lipofection, vescicle fusion, and other techniques known by those skilled in the art.

The present invention will now be described with reference to the following specific, non-limiting comparative examples.

### Example 1

### Glycosylation of hTLR3 Extracellular Domain

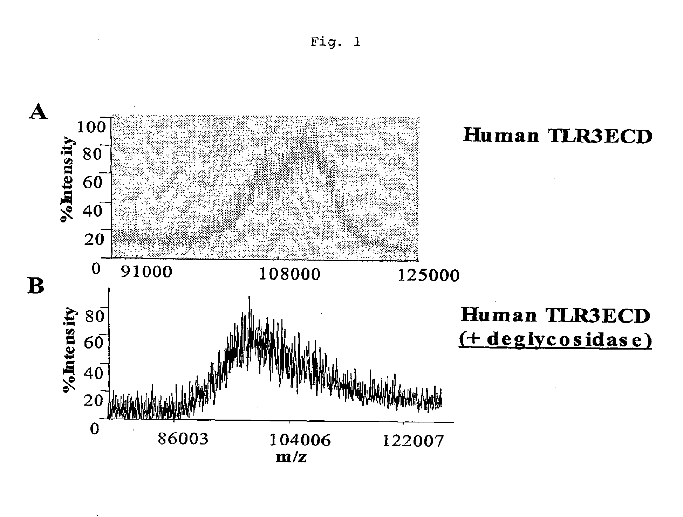

Mass spectroscopic analysis performed on the purified, soluble ECD of hTLR3 recombinantly expressed in *Homo sapiens* derived HEK293 cells (ATCC® number: CRL-1573™) revealed a high degree of charge heterogeneity among ionized hTLR3 ECD fragment species (Fig. 1A) and an average ion fragment mass of 110 kD. Treatment of recombinantly expressed hTLR3 ECD with a mixture of deglycosidases specific for O-linked and N-linked glycosylation followed by mass spectroscopic analysis revealed that deglycosidase treatment decreased the average ion fragment mass to 94 kD (Fig. 1B). Additionally, incubation of hTLR3 ECD with N-acetylneuraminic acid (NANAse), O-glycosidase DS, peptide N-Glycosidase F (PNGase F), or a cocktail containing all of these enzymes decreased glycoprotein specific staining of SDS-PAGE resolved hTLR3 ECD. Together these results indicate that the hTLR3 ECD is both N- and O-glycosylated.

hTLR3 ECD for mass spectroscopic and glycoprotein specific SDS-PAGE analyses were prepared as follows. First, a cDNA (SEQ ID NO: 1) encoding the full-length *Homo sapiens* TLR3 protein (SEQ ID NO: 2) and identical to accession number U88879 was cloned into pcDNA3.1. Next, a cDNA fragment encoding the hTLR3 ECD (SEQ ID NO: 3) and consisting of amino acids 1-703 of full length hTLR3 (SEQ ID NO: 4) was cloned into pcDNA3.1. This cDNA fragment was cloned into pcDNA3.1 in frame and on the 5' side of a cDNA encoding a hexahistidine affinity tag. The resulting plasmid encodes a recombinant hTLR3 ECD comprising a C-terminal hexahistidine tag.

hTLR3 ECD encoded by this plasmid was expressed, processed, and secreted by transiently transfected HEK293 cells. Cells were transfected and cultured using standard methods. The recombinant hTLR3 ECD with carboxy terminal hexahistidine affinity tag was purified from cell culture supernatant using a Ni-NTA resin and was further purified by ion exchange chromatography using standard methods. The majority of hTLR3 ECD protein produced by this process is predicted to lack the first 26 amino terminal, signal peptide residues due to post-translational proteolytic processing and secretion. The hTLR3 ECD protein purified comprised amino acid residues 27 to 703 (SEQ ID NO: 6) of full length hTLR3 (SEQ ID NO: 2).

Mass spectroscopic analyses of purified, recombinant hTLR3 ECD were performed as follows. First, samples were concentrated with a Nanosep™ centrifugation concentrator (Pall Corp., East Hills, NY), desalted using C-18 Zip Tips (Millipore Corp., Billerica, MA), and eluted with 50% acetonitrile/0.1% trifluoroacetic acid. 1 µL of each sample was then co-crystallized with the matrix in 2,5-dihydroxybenzoic acid in acetonitrile/water (50:50) containing 0.1% trifluoroacetic acid (TFA). MALDI-TOF experiments were then performed using standard methods and recorded using an ABI Voyager-DE™ STR mass spectrometer. To examine glycosylation, 1 µg of the purified hTLR3 ECD protein was incubated with a mixture of N-glycanase, O-glycanase, and sialidase at 37°C for 24 h. Products of this incubation were then analyzed by MALDI-TOF spectroscopy as described above.

Preparation of purified hTLR3 ECD for SDS-PAGE resolution and glycoprotein specific visualization was performed as follows. First, samples containing recombinantly expressed hTLR3 ECD alone, hTLR3 ECD incubated with N-acetylneuraminic acid (NANAse), hTLR3 ECD incubated with O-glycosidase DS, peptide N-Glycosidase F (PNGase F), or a cocktail containing all of these enzymes and hTLR3 ECD were prepared. Samples containing equal amounts of hTLR3 ECD were then resolved by SDS-PAGE on a 4-12% gradient gel using standard methods and glycoprotein was visualized with the glycoprotein specific SYPRO Ruby protein gel stain (Invitrogen, Inc., Carlsbad, CA).

### Example 2

### Effect of N-Glycosylation on Poly(I:C) Induced Activation of hTLR3 Signaling in Cells

In these experiments, TLR3 signaling was assayed using the pNF-κB-Luciferase (Stratagene, Inc., Carlsbad, CA) reporter gene construct transiently transfected by standard methods into HEK293 cells. This reporter construct comprised an NF-κB responsive DNA element linked to a luciferase reporter gene. Activation of TLR3 by poly(I:C) ligand increases NF-κB activity and results in activation of NF-κB responsive genes such as the luciferase reporter gene. HEK293 cells transfected with pNF-FCB-Luciferase were transiently co-transfected with the control vector pHRL-TK constitutively producing a luciferase protein derived from *Renilla.* A cDNA encoding full-length hTLR3 was also transiently co-transfected, using standard methods, into HEK-293 cells transfected with the luciferase reporter gene.

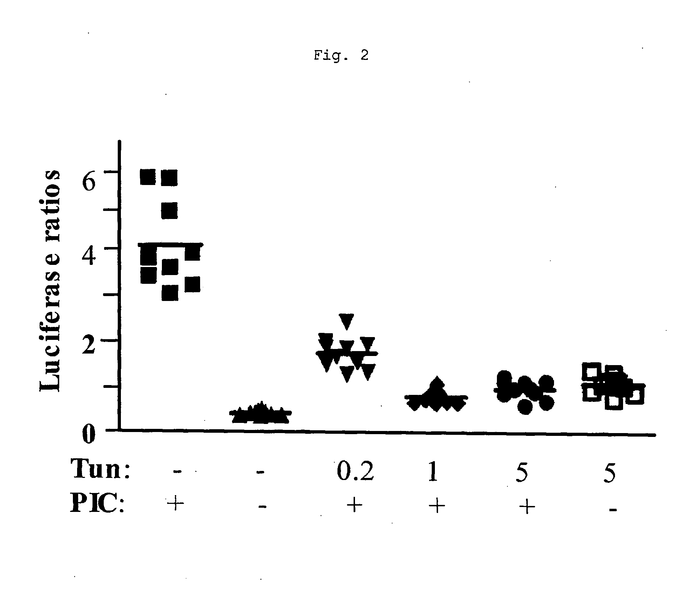

After transfection, cells were treated with non-toxic doses of tunicamycin as shown in Fig. 2. Tunicamycin inhibits N-linked glycosylation by inhibiting the attachment of N-acetylglucoasamine which is the first sugar residue attached to a peptide chain during glycosylation. Treated or control HEK293 cells were then incubated with 10 µg/ml of the hTLR3 ligand poly(I:C) (PIC) as indicated in Fig. 2.

After treatment, luciferase expressed from pNF-κB-Luciferase and pHRL-TK was assayed using standard methods. Data was expressed as a "luciferase ratio" equal to the pNF-κB-Luciferase activity normalized to pHRL-TK luciferase activity. Results are presented as scatter plots of data collected from 9 individual samples and is representative of 3 independently conducted, identical experiments.

### Example 3

### Effect of hTLR3 ECD N-Glycosylation Inhibition on Poly(I:C) Induced Activation of hTLR3 Signaling in Cells

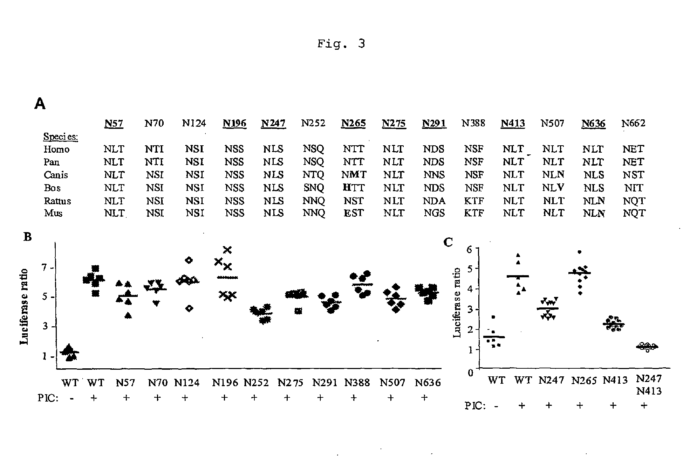

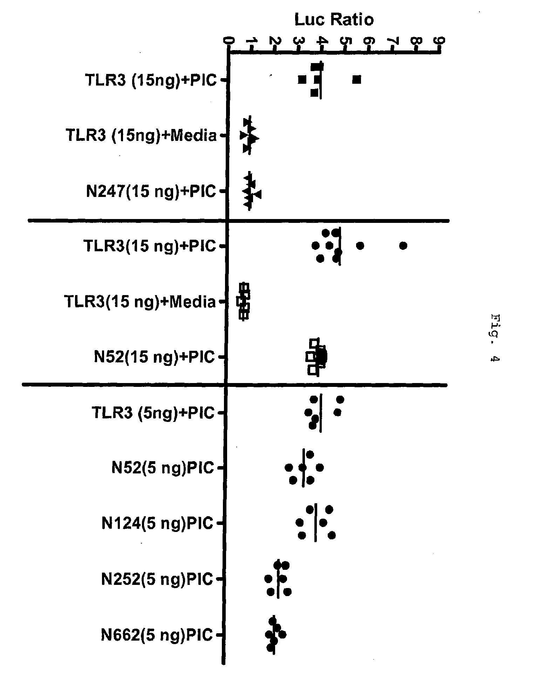

Surprisingly, two potential N-glycosylation sites, N247 and N413, of the many possible sites within the hTLR3 sequence of SEQ ID NO: 2 were found to play a critical role in hTLR3 signaling (Fig. 3C). Additionally, two other potential N-glycosylation sites in SEQ ID NO: 2, N252 and N662, were also found to play an important role in hTLR signaling (Fig. 3B and Fig. 4). Positions N247, N252, N413, and N662 in SEQ ID NO: 2 are respectively equivalent to N221, N226, N387, and N636 of SEQ ID NO: 6.

The hTLR3 ECD has several potential N-linked glycosylation sites, based on the presence of the N-glycosylation motif Asn-X-Ser/Thr (Fig. 3A) in various TLR3 homologs. Only five of these potential N-linked glycosylation sites were conserved between *Homo sapiens, Pan troglodytes, Canis familiaris, Bos taurus, Rattus norvegicus* and *Mus musculus* TLR3 homologs (Fig. 3A) as assessed by standard CLUSTALW alignment. The five conserved sites in SEQ ID NO: 2 were N57, N196, N247, N275, and N413. Four other potential N-glycosylation sites in SEQ ID NO: 2 varied between the various TLR3 homologs examined (Fig. 3A). These four other potential, albeit non-conserved, N-glycosylation sites were N252, N265, N291, N507, N636, and N662 of SEQ ID NO: 2.

The asparagines residues within these potential N-linked glycosylation sites in the hTLR3 ECD were individually mutated using standard methods to alanine residues (Fig. 3 and Fig. 4). Four additional asparagine residues present in the hTLR3 ECD that do not contain a match to the Asn-X-Ser/Thr N-glycosylation motif in any TLR3 homolog were also mutated as a control. These residues were N70, N124, and N388 of full length hTLR3 (SEQ ID NO: 2). All mutagenesis was performed on cDNAs encoding full-length hTLR3 in order to produce full-length, processed, and secreted hTLR3 with a mutagenized ECD. Mutated positions in SEQ ID NO: 2 are indicated in the figures by identification of the position of the alanine substitution (*e.g*. N70 means N70 has been replaced with an alanine residue).

Plasmids encoding and expressing the mutant hTLR3 cDNA constructs described above were individually transfected into HEK293T cells. Cells transfected with plasmids encoding individual, mutant hTLR3 molecules were also simultaneously transfected with the pNF-κB-Luciferase reporter and pHRL-TK luciferase control vector. Transfections and cell culture were performed using standard methods.

Transfected and control HEK293 cells were then incubated with 10 µg/ml (Fig. 3B and Fig. 4) or 2.5 µg/ml (Fig. 3C) of the hTLR3 ligand poly(I:C) (PIC) as indicated to assess the effect of the various hTLR3 mutations on hTLR3 signaling. After treatment luciferase expressed from pNF-κB-Luciferase and pHRL-TK was assayed using standard methods. Data was expressed as a "luciferase ratio" as described above. Results are presented as scatter plots of data collected from 6 individual samples.

## Claims

1. A peptide chain comprising the amino acid sequence of SEQ ID NO: 6 and at least one mutation within 3 amino acid residues of a position aligning to N636 of the amino acid sequence SEQ ID NO: 6.

2. A peptide chain comprising the amino acid sequence, SEQ ID NO: 14, SEQ ID NO: 18, or SEQ ID NO: 20.

3. A nucleic acid encoding the peptide chain of claim 1 or 2.

4. The nucleic acid of claim 3 comprising the nucleic acid sequence of, SEQ ID NO: 13, SEQ ID NO: 17, or SEQ ID NO: 19.

5. The peptide chain of claim 1 or 2 for use in a method of modulating TLR3 activity in a cell, wherein the modulation is attenuation.

6. The nucleic acid of claim 3 for use in a method of modulating TLR3 activity in a cell, wherein the modulation is attenuation.

## Patentansprüche

1. Peptidkette, umfassend die Aminosäuresequenz der SEQ ID NO: 6 und wenigstens eine Mutation innerhalb von 3 Aminosäureresten einer sich an N636 der Aminosäuresequenz SEQ ID NO: 6 ausrichtenden Position.

2. Peptidkette, umfassend die Aminosäuresequenz SEQ ID NO: 14, SEQ ID NO: 18 oder SEQ ID NO: 20.

3. Nukleinsäure, codierend für die Peptidkette nach Anspruch 1 oder 2.

4. Nukleinsäure nach Anspruch 3, umfassend die Nukleinsäuresequenz SEQ ID NO: 13, SEQ ID NO: 17 oder SEQ ID NO: 19.

5. Peptidkette nach Anspruch 1 oder 2 zur Verwendung in einem Verfahren zur Modulation der TLR3-Aktivität in einer Zelle, wobei es sich bei der Modulation um Abschwächung handelt.

6. Nukleinsäure nach Anspruch 3 zur Verwendung in einem Verfahren zur Modulation der TLR3-Aktivität in einer Zelle, wobei es sich bei der Modulation um Abschwächung handelt.

## Revendications

1. Chaîne peptidique comprenant la séquence d'acides aminés de SEQ ID NO : 6 et au moins une mutation au sein de 3 résidus d'acides aminés d'une position s'alignant avec N636 de la séquence d'acides aminés SEQ ID NO : 6.

2. Chaîne peptidique comprenant la séquence d'acides aminés SEQ ID NO: 14, SEQ ID NO: 18, ou SEQ ID NO : 20.

3. Acide nucléique codant pour la chaîne peptidique de la revendication 1 ou 2.

4. Acide nucléique de la revendication 3, comprenant la séquence d'acides nucléiques de SEQ ID NO : 13, SEQ ID NO : 17, ou SEQ ID NO : 19.

5. Chaîne peptidique de la revendication 1 ou 2, destinée à être utilisée dans une méthode de modulation de l'activité de TLR3 dans une cellule, la modulation étant une atténuation.

6. Acide nucléique de la revendication 3, destiné à être utilisé dans une méthode de modulation de l'activité de TLR3 dans une cellule, la modulation étant une atténuation.
